(19) Europäisches Patentamt
European Patent Office
Office européen des brevets

(11) Publication number: 0 387 830 A2

(12) **EUROPEAN PATENT APPLICATION**

(21) Application number: 90104809.0

(22) Date of filing: 14.03.90

(51) Int. Cl.5: C07H 15/252, C12N 1/20, C12P 19/56, A61K 31/70, //(C12N1/20,C12R1:365), (C12P19/56,C12R1:365)

The applicant has filed a statement in accordance with Rule 28 (4) EPC (issue of a sample only to an expert). Accession number(s) of the deposit(s): FERM - 10615 and FERM - 2737.

(30) Priority: 15.03.89 JP 64667/89
01.11.89 JP 285462/89

(43) Date of publication of application: 19.09.90 Bulletin 90/38

(84) Designated Contracting States: AT BE CH DE DK ES FR GB GR IT LI LU NL SE

(71) Applicant: YAMANOUCHI PHARMACEUTICAL CO. LTD.
No. 3-11 Nihonbashi-Honcho, 2-chome
Chuo-ku
Tokyo(JP)

(72) Inventor: Morioka, Motoo
3-12-1, Atago
Niiza-shi, Saitama 352(JP)
Inventor: Sasaki, Toshio
5-22-12-504, Buzou
Urawa-shi, Saitama 336(JP)
Inventor: Suzuki, Ken-ichi
1-67, Futatsuya
Kitamoto-shi, Saitama 364(JP)
Inventor: Nagai, Kouji
3-16-1, hasune, Itabashi-ku
Tokyo 174(JP)
Inventor: Yamaguchi, Hiroshi
136-51, 10-205 Nara-cho
Ohmiya-shi, Saitama 330(JP)
Inventor: Nohara, Chieko
4-9-23 Kashiwa-cho
Shiki-shi, Saitama 353(JP)
Inventor: Tokunaga, Tatsuhiro
2-8-508 Inodanchi
Toride-shi, Ibaraki 302(JP)
Inventor: Hiramoto, Masashi
134 Aza-maeda, Gumyoji-cho, Minami-ku
Yokohama-shi, Kanagawa 232(JP)
Inventor: Takebayashi, Yukihiro
3-3-2, Wakamiya
Ichikawa-shi, Chiba 272(JP)

(74) Representative: Behrens, Dieter, Dr.-Ing. et al
Wuesthoff & Wuesthoff Patent- und Rechtsanwälte Schweigerstrasse 2
D-8000 München 90(DE)

(54) **The anthracycline substances, microorganisms producing the same and a process for preparing the substances.**

(57) Anthracycline substances (I) represented by the following plane chemical formula:



Xerox Copy Centre

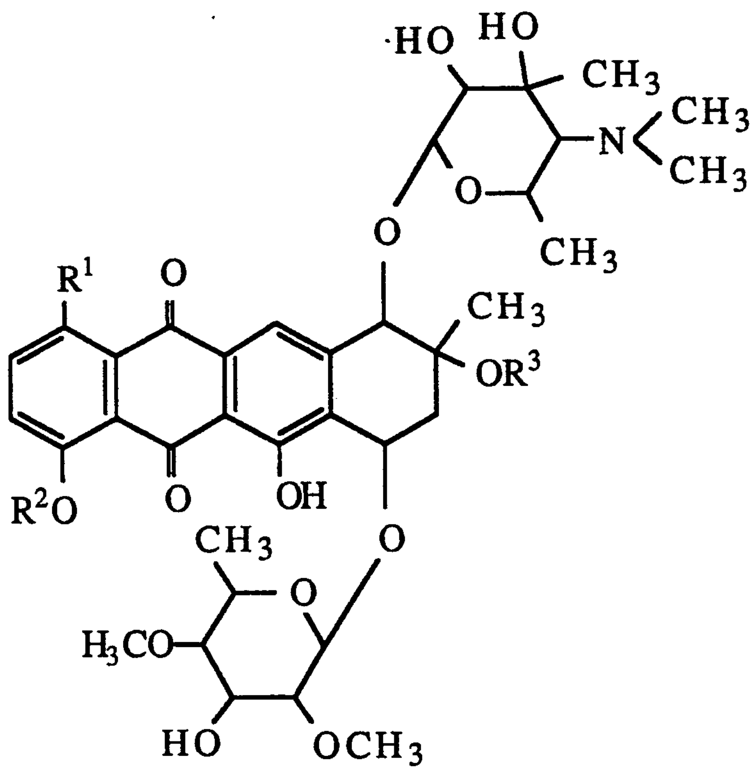

(I)

wherein $R^1$ represents a hydrogen atom or a hydroxyl group, $R^2$ represents a hydrogen atom or a lower alkyl group and $R^3$ represents a hydrogen atom or a lower alkanoyl group
or acid addition salts thereof.

# The anthracycline substances, microorganisms producing the same and a process for preparing the substances

## Background of the Invention

### Field of the Invention

The present invention relates to novel anthracycline substances which are useful as medicament or drugs, particularly antitumor agents, microorganisms producing the same and a fermentation process for producing the substances.

In the course of studies on substances produced by a variety of microorganisms existing in the nature, the present inventors found such a fact that novel anthracycline substances having a strong antitumor activity are produced in a culture medium in which a microorganism which belongs to genus Nocardia and which has an ability to produce the anthracycline substances is cultured. The present invention was completed by isolating the substances.

### Description of the related art

A variety of anthracycline compounds which are produced by microorganisms are known. Some of the compounds such as daunomycin, adriamycin and aclacinomycin are used effectively in clinical applications. However, when drugs containing these compounds are administrated, in almost all cases, tumorious target cells gain a resistance to the drugs. The present inventors found that the novel anthracycline substances according to the present invention are effective to the animal tumorious cells which have gained the resistance to known anthracycline compounds, as is clarified hereinafter.

Accordingly, an object of the present invention is to provide novel anthracycline substances having an effective antitumor activity.

Another object of the present invention is to provide a novel process for preparing the antitumor substances.

A still another object of the present invention is to provide a novel strain or a mutant thereof which has an ability to produce the anthracycline substances.

## Summary of the Invention

The present invention provides anthracycline substances (I) represented by the following plain formula:

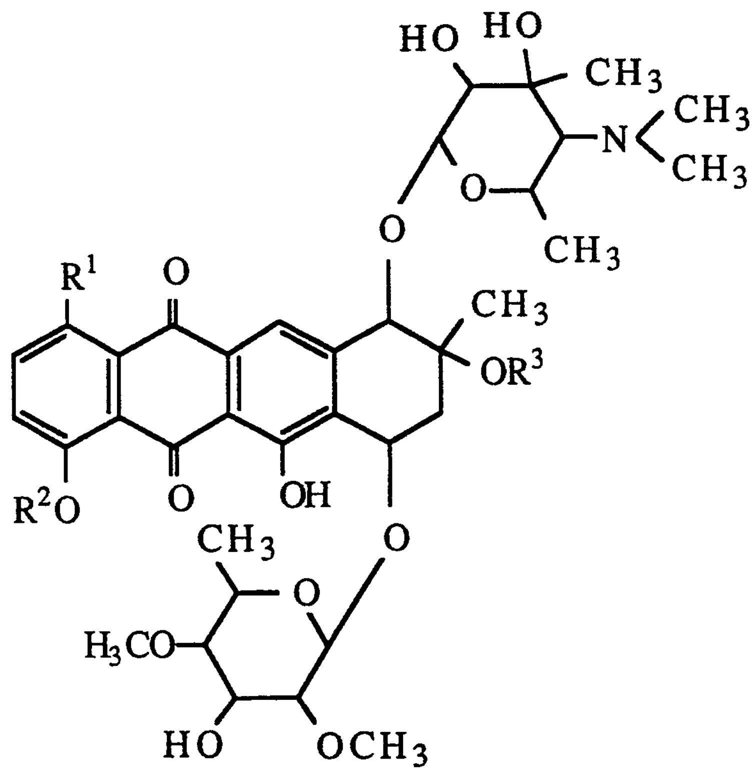

(I)

wherein $R^1$ represents a hydrogen atom or a hydroxyl group, $R^2$ represents a hydrogen atom or a lower alkyl group and $R^3$ represents a hydrogen atom or a lower alkanoyl group,
or their acid added salts.

A lower alkyl or alkanoyl group means an alkyl or alkanoyl group having preferably 1 to 6, especially 1 to 4, carbon atoms.

The substances (I) according to the present invention possess glucose chains at 7- and 10-position of the anthracycline skeleton. A characteristic chemical structure of the compounds resides in that a dimethylamino group which did not exist in known anthracycline antibiotics exists at 4-position of the glucose chain which is bonded to 10-position.

The present invention also provides a process for preparing the anthracycline substances comprising cultivating a microorganism which belongs to genus Nocardia and which has an ability to produce the anthracycline substances (I) in a culture medium so that the substances are produced in a fermentation broth and collecting the substances produced.

The present invention also provides a novel microorganism or a mutant thereof which belongs to genus Nocardia and which has the ability to produce the anthracycline substances (I).

Now, the microorganism according to the present invention, the process for producing the compounds (I) according to the present invention and the substances (I) obtained will be described in details in this order.

A strain which can produce the novel substances having an antitumor activity according to the present invention is a microorganism which was isolated from a soil sample collected in Indonesia. The microbiological properties of this strain (YL-01641P strain) are as follows:

(i) Morphological properties

The substratal mycelium grows well and branches off in a variety of organic and inorganic mediums and has a width of about 4 μm. Concresence of the mycelia is observed in a later stage of fermentation and a small cleavage is observed on a substratal mycelium in certain media. Aerial hypha branches irregularly in a form of *rectus flexibilis* and forms a relatively short spore-bearing hypha. On and after the 5th to 7th weeks' culture of 5 to 10 spore chains are formed in the vicinity of a front end of the spore-bearing hypha. The front end has a coiled shape. In some cases, a long spore chain branched from a main aerial hypha is observed. In this case, quasi-sporangium structure having a shape of a bundle or a loop of hyphae is also

observed. The spore has a cylindrical shape and has a size of 1.8 to 1.2 μm × 0.5 to 0.8 μm and a smooth surface. No special organ such as sporangium, sclerotium, motile element or verticillate is observed.

(ii) Properties on various agar culture media

The properties of the strain on various agar culture media are shown in Table 1. Unless otherwise mentioned, the strain was cultivated at 28 °C for 21 days and observed according to the conventional method. The expression of color depends on "Guide to Color Standard" (Japan Color Institute).

Table 1

| Medium | Growth (G) | Aerial Hyphae (A) | Color on a reverse side (R) | Soluble Pigment (S) |
|---|---|---|---|---|
| Sucrose nitrate agar | Fair | Well, Light-orange | Light-brown | None |
| Glucose asparagine agar | Fair | Well, Light-orange | Light-orange | None |
| Glycerine asparagine agar (ISP-5) | Fair | Fair, Light-orange | Light yellow-orange to dark-orange | None |
| Inorganic salts-starch agar (ISP-4) | Fair | Fair, Light-orange | Light yellow-orange | None |
| Tyrosine agar (ISP-7) | Fair | Fair, Light-orange | Light-orange to dark-orange | None |
| Nutrient agar | Fair | A little poor, Light yellow-orange | Dark yellow-orange | None |
| Yeast extract-malt extract agar (ISP-2) | Well | Well, Light-orange | Dark yellow-orange | None |
| Oatmeal agar (ISP-3) | Well | Well, Light-yellow orange | Light yellow-brown to dark yellow-orange | None |
| Bennett's agar | Well | Well, Light-orange | Dark-yellow to dark yellow-orange | None |
| Tripton yeast extract agar | Fair | Not grow | Dark yellow-orange | None |
| (Note) G: Growth | | | | |
| A: Growth of aerial hyphae and their color | | | | |
| R: Color on a reverse side | | | | |
| S: Soluble pigments | | | | |

(iii) Physiological characteristics of the strain

(1) Range of growth temperature: 20 ~ 40 °C
Optimum growth temperature: 27 ~ 32 °C
(2) Liquefaction of gelatin

Gelatin only (20 °C): negative
Glucose peptone gelatin (28 °C): negative
(3) Coagulation of skim milk: positive
Peptonization of skim milk: positive
(4) Reduction of nitrate: positive
(5) Hydrolysis of starch: negative
(6) Production of melanin pigment in trypton-yeast extract medium: negative
in tyrosine agar medium: negative
in peptone-yeast extract-ion agar: negative

(Note)

The growth temperatures (5, 10, 15, 20, 25, 28, 30,37,40, 45 and 50 °C) show the results of observation for a period from 7th to 21st day and the effects on skim milk show the results of observation for a period from 3rd to 21st day at 37 °C. The other results were obtained from observation at 28 °C after two weeks unless otherwise mentioned.

(iv) Utilization of carbon sources

(cultured at 28 °C in a Pridham-Gottlieb agar medium)
L-arabinose - Mannose -
D-xylose - Melibiose -
D-glucose + Lactose -
D-fructose + D-galactose +
Sucrose + Maltose +
Inositol + Salicin -
Rhamnose - Trehalose +
Raffinose - Glycerine +
D-mannitol + Dextrin +
Starch + Xanthine -
(Note) "+" indicates good growth, and
"-" indicates no growth

(v) Chemical analysis of the strain components

Analysis of products obtained by acid hydrolysis of the strain and of cell wall components was effected according to a method of LECHVALIER (LECHVALIER, M.P. et al.; pp 277-238 in DIETZ, A et al, Actionomycete Taxonomy, SIM Special publication No. 6, 1980). The results revealed that the cell wall type is IV-4 as is shown in Table 2.

Table 2

| | |
|---|---|
| Cell wall amino acid: | Meso-diaminopimelic acid, Alanine, Glutamic acid |
| Whole cell sugar pattern: | Arabinose, Galactose |
| Menaquinone type: | MK-8 |
| Phospholipid: | PII (containing phosphatidylethanolamine) |
| The presence of mycolic acid: | positive |
| Content of GC: | 66 % |

The above-mentioned properties are summarized as following: The strain YL-01641P forms a spore chain on a short spore-bearing hyphae which is formed on an aerial hyphae. In a later stage of cultivation, a fragmented spore chain is observed in a main axis of the aerial hyphae. The aerial hyphae sometimes forms a bundle and/or a coiled structure. Quasi-sporangium structure can be observed. A cleavage may occur even at a substrate hyphae in a later stage of cultivation. The cell wall type is classified to IV-A type

and the basic menaquinone type is MK-8 (H4, H2).

From literature search for actinomycetes, it is concluded that the strain belongs to sporoactinomyces (actinomycetes which produce spore chains). In known genus which satisfy the results of the chemical analysis shown in Table 2 and the morphological properties, genus Nocardia is mentioned.

The genus Nocardia had been classified in three types according to their shapes in an early stage of fermentation, existence or absence of the aerial hyphae ("Bergey's manual of determinative bacteriology" the 8th edition, 1974) but most of Nocardia which had belonged to types I and II were transferred to the genus Rhodococcus. And hence, it is requested to establish another new classification from a completely different point of view.

"Bergey's manual of Systematic Bacteriology" (vol. 2, 1986) discloses nine species which belong to the genus Nocardia but none of them corresponds to the strain YL-01641. Accordingly, the strain typifies a new species which belongs to the genus Nocardia and was named Nocardia sp. YL-01641P.

Nocardia sp. YL-01641P has been deposited with Fermentation Research Institute under accession number FERM-10615 on March 9, 1989 and transferred to accession number FERM BP-2737 on January 19, 1990.

In the process for producing the anthracycline substances according to the present invention, the strain of Nocardia sp. YL-01641P is cultivated in a nutrient medium under an aerobic condition to obtain a fermentation broth containing the anthracycline substances according to the present invention. Known nutrients for actinomycetes can be used. Examples of nitrogen sources are commercially available peptone, meat extract, corn steep liquor, cottonseed meal, peanut meal, soybean meal, yeast extract, NZ-amine, hydrolyzed casein, fish meal, sodium nitride and ammonium nitride. Examples of carbon sources are commercially available carbohydrates such as glycerin, sucrose, starch, glucose, maltose, and molasse, and fats. Inorganic salts such as sodium chloride, phosphates, calcium carbonate or magnesium sulfide may be added to the medium. Any other substance which is utilized by the organism and is useful for production of the anthracycline substances according to the present invention can be used.

The fermentation can be carried out by the same process as usual process used for producing antibiotics and may be effected in a solid medium or in a liquid medium. In the case of liquid medium, the fermentation can be carried out by rotary shaker, jar fermentater, tank or stationary fermentation methods, preferably by aeration fermentation under agitation. The fermentation temperature can vary in a range from 15 to 40 °C where the organism can grow and produces the substances according to the present invention and preferably from about 25 to 32 °C. The pH of the medium varies from about 4 to 7 and preferably from 6 to 8. The fermentation duration depends on various conditions and is usually from 10 to 168 hours. The maximum producing activity of the objective substances is attained usually in about 24 to 120 hours in this fermentation process.

The objective compound can be isolated from the fermentation broth by means of extraction, separation and purification which are adopted usually for isolating objective compounds from metabolites produced by microorganisms in fermentation broths. The objective substances in the fermentation broth are extracted directly from the fermentation broth or are extracted from a centrifuged and/or filtrated broth with an organic solvent which is immiscible with water such as ethylacetate, chloroform, benzene, toluene etc. The object substances can be extracted by contacting the filtrate of fermentation liquid with a suitable support to adsorb the objective substances contained in the filtrate and then eluting them with proper solvents. In more details, the object substances are adsorbed by contact with high-porous adsorbent resins such as Amberlite XAD-2, Diaion HP-20, Diaion SP-900 or Diaion CHP-20P or ion exchange resins such as Dowex 50-W, Amberlite IRC-50. Then, fractions containing the objective substances are eluted with a mixed solvent of water and organic solvent(s) such as methanol, ethanol, acetone, acetonitrile for the case of adsorbent resins and with an aqueous solution of hydrochloric acid or sulfuric acid for the case of ion exchange resins. When the extraction is effected with such organic solvents as ethylacetate or butylacetate, the solvent is added to the filtrate of fermentation broth. After the resulting mixture is shaken well to extract the objective substances, the obtained extract is reverse-extracted with acidic water to dissolved the objective substances into water. After the acidic water is neutralized, it is reverse-extracted with an organic solvent. By repeating these procedures, fractions containing the objective substances in a higher proportion can be obtained. The fractions containing the objective substances obtained by one of above-mentioned procedures can be separated much finely by usual methods including adsorption techniques such as column chromatography or thin layer chromatography using, as a support, active carbon, alumina, silica gel, cellulose etc or high performance liquid chromatography using a column of ODS reverse layer support. The objective substances obtained by these methods are purified by crystallization from a solvent such as methanol or ethylacetate. The novel anthracycline substances produced in the fermentation broth may be isolated in a form of free bases. These free bases can be reacted with inorganic acid such as hydrochloric acid, sulfuric

acid, phosphoric acid or with organic acid such as formic acid, acetic acid, citric acid, tartaric acid, p-toluene sulfonic acid by usual method so that the free bases are changed to corresponding salts.

## Brief Description of the Drawing

Figure 1 is an ultraviolet absorption spectrum of a compound YL-01641P-$A_1$,
Figure 2 is $^1$H-NMR spectrum of the compound YL-01641P-$A_1$,
Figure 3 is $^{13}$C-NMR spectrum of the compound YL-01641P-$A_1$,
Figure 4 is an ultraviolet absorption spectrum of a compound YL-01641P-$A_2$,
Figure 5 is $^1$H-NMR spectrum of the compound YL-01641P-$A_2$,
Figure 6 is an ultraviolet absorption spectrum of a compound YL-01641P-$B_2$,
Figure 7 is $^1$H-NMR spectrum of the compound YL-01641P-$B_2$,
Figure 8 is an ultraviolet absorption spectrum of a compound YL-01641P-$B_1$,
Figure 9 is $^1$H-NMR spectrum of the compound YL-01641P-$B_1$,
Figure 10 is $^{13}$C-NMR spectrum of the compound YL-01641P-$B_1$,
Figure 11 is an ultraviolet absorption spectrum of a compound YL-01641P-$C_1$ and
Figure 12 is $^1$H-NMR spectrum of the compound YL-01641P-$C_1$.

The following examples are given solely for the purpose of illustration and are not to be constructed as limitation of the present invention.

## Example 1

### (a) Process for producing substance YL-01641P-$A_1$

A culture medium (pH of 8.0) containing 2.0 % of dextrine, 0.5 % of corn steep liquor, 0.5 % of polypeptone, 0.5 % of yeast extract, 0.3 % of meat extract, 0.52 % of brain heart infusion bouillon and 0.5 % of calcium carbonate was prepared. The resulting culture medium was divided into a plurality of 500 mℓ Erlenmeyer flasks each containing 60 mℓ of the culture medium and then sterilized at 120 °C for 20 minutes. Hyphae of Nocardia sp. YL-01641P which had grown well on Bennett's agar medium was inoculated in the culture medium which was then incubated under shaking at 27 °C for 48 hours to prepare a seed culture.

A fermentation medium (pH 7.0) containing 3.0 % of potato starch, 1.0 % of roasted wheat germ, 1.0 % of copra meal, 0.2 % of feather meal and 0.5 % of calcium carbonate was prepared. The resulting culture medium was divided into a plurality of 500 mℓ Erlenmeyer flasks each containing 60 mℓ of the culture medium and was sterilized at 120 °C for 20 minutes.

Then, 3.0 % of the seed culture was inoculated in the fermentation medium and was incubated at 28 °C for 96 hours. Into 1ℓ of a fermentation broth thus obtained, Radio Light # 600 (a product of Showa Chemical Industries Co., Ltd.) was added. After agitation, the resulting mixture was filtered to obtain 650 mℓ of filtrate of the fermentation broth. After this filtrate was adjusted to pH 7.0, 500 mℓ of ethylacetate was added twice to the filtrate and extracted. After the extract was concentrated under reduced pressure to 100 mℓ, 100 mℓ of water was added. Water layer was adjusted to pH 2.5 with 1-N hydrochloric acid and was shaken satisfactorily so that the objective substance was reverse-extracted from a solvent layer into a water layer. Into the resulting acidic extractant, 50 mℓ of ethylacetate was added and the water layer was adjusted to pH 7.5 with 1-N sodium hydroxide and shaken before re-extraction. The above-mentioned procedures were repeated twice to obtain totally 200 mℓ of ethylacetate extractant containing YL-06141P-$A_1$ substance. This extract was dried over anhydrous sodium sulfate. After anhydrous sodium sulfate was removed by filtration, the resulting filtrate was concentrated under reduced pressure to obtain 3 mℓ of a concentrated solution of crude YL-01641P-$A_1$ substance.

The presence of the objective substance YL-01641P-$A_1$ was confirmed by thin layer chromatography using silica gel plate (Kieser Gel $60F_{254}$, 20 cm x 20 cm, a product of Merk) which was developed with chloroform / methanol (90 : 10) (Rf = 0.38).

In order to purify the crude YL-01641P-$A_1$ substance contained in the concentrated solution, the solution was charged on three silica gel plates (Kieser Gel $60F_{254}$, 20 cm x 20 cm, product of Merk) at a

height of 2 cm from the bottom in a form of a band and was developed to a height of 15 cm with chloroform/methanol (90 : 10). After drying in air, a red-orange band (Rf = 0.38) which shows the presence of YL-01641P-$A_1$ substance was confirmed under a UV lamp and visible lights. This part was scraped and eluted with a solvent of chloroform/methanol (90 : 10). The resulting solution was concentrated to dryness under reduced pressure. Then, the solid was dissolved with a small amount of methanol under heat. The resulting solution was left in a refrigerator to obtain needle-like crystals. After the solvent was removed, the crystals were dried in a desiccator in vacuo overnight to obtain 2.15 mg of pure YL-01641P-$A_1$ substance.

The resulting free base of YL-01641P-$A_1$ substance have following physicochemical properties:

(1) Appearance: red needle-like crystal

(2) Nature: basic

(3) Solubility in solvents: soluble in methanol, ethanol, acetone, ethylacetate, benzene, toluene and chloroform but sparingly soluble in water and substantially insoluble in hexane.

(4) Ultraviolet absorption spectrum: an ultraviolet absorption spectrum of YL-01641P-$A_1$ substance measured in methanol is shown in Figure 1.

(5) Mass spectrum (FAB): 790 (M + 1)

(6) Molecular weight: 789.831

(7) Molecular formula: $C_{39}H_{51}NO_{16}$

| (8) Elemental analysis (as $C_{39}H_{51}N_{16}\cdot 0.7H_2O$) | | | |
|---|---|---|---|
| | C (%) | H (%) | N (%) |
| Found | 58.30 | 6.57 | 1.68 |
| Calculated | 58.38 | 6.58 | 1.75 |

(9) $^1$H-NMR Spectrum: $^1$H-NMR Spectrum (500 MHz, $CDCl_3$) of YL-01641P-$A_1$ substance is shown in Figure 2.

(10) $^{13}$C-NMR Spectrum: $^{13}$C-NMR Spectrum (125 MHz, $CDCl_3$) of YL-01641P-$A_1$ substance is shown in Figure 3.

From these physicochemical properties, YL-01641P-$A_1$ substance is represented by the following formula:

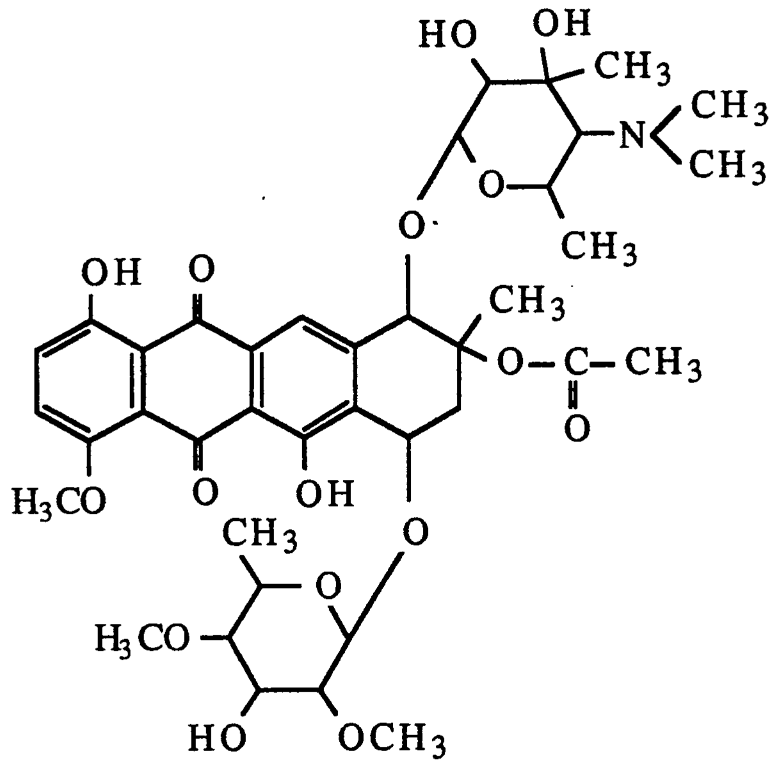

(b) Process for preparing hydrochloric acid salt

To 25.6 mg of purified YL-01641P-$A_1$ substance obtained by the process (a), a small amount of water was added and then 0.5 N-hydrochloric acid was added carefully drop by drop under stirring to dissolve YL-01641P-$A_1$ substance. This procedure was continued until the substance was completely dissolved. The resulting aqueous solution of YL-01641P-$A_1$ hydrochloric acid salt was lyophilized to give 26.76 mg of a red powder of YL-01641P-$A_1$ substance which is easily soluble in water. The solubility in water of this powder is remarkably higher than that of the YL-01641P-$A_1$ base.

Example 2

Into 4 ℓ of a fermentation broth of the strain YL-01641P which was prepared by the same method as Example 1, sulfuric acid was added to adjust pH to 2.0 and then stirred. Then, Radio Light # 600 (product of Showa Chemical Ind. Co., Ltd.) was added to the broth, stirred and then filtered out. The filtrate was adjusted to pH 5.02 to precipitate insoluble substances which was then separated by filtration to obtain 2.98 ℓ of a clear filtrate of fermentation broth. The filtrate was applied to a column (60 mℓ, an outer diameter of 2.3 cm, a length of 19 cm) of Diaion HP-20 (a product of Mitsubishi Chemical Industries, Ltd.) so that the objective substance YL-01641P-$A_1$ is adsorbed. The column was washed with 1 ℓ of distilled water and was eluted successively with each 120 mℓ of 30 %, 40 %, 50 %, 60 % and 70 % aqueous acetone solutions prepared with 0.01 M phosphate buffer. The elution was effected step-wise and started with an acetone solution of lower concentration. YL-01641P-$A_1$ substance was eluted in fractions of 40 % and 50 % acetone solutions.

The presence of YL-01641P-$A_1$ in the eluted liquids was confirmed by thin layer chromatography using silica gel plates (Kieser Gel, $60F_{254}$, 20 cm × 20 cm, a product of Merk) which was developed with chloroform-methanol (90 : 10).

Then, the eluted fractions were concentrated under reduced pressure to give 107.2 mg of a red crude powder of YL-01641P-$A_1$ substance (purity : 34.1 %). The crude powder of YL-01641P-$A_1$ substance was dissolved in 100 mℓ of ethylacetate. After insoluble precipitates were filtered out , 100 mℓ of water was

added to a filtrate. Then, hydrochloric acid was added under shaking to a water layer to adjusted pH to 2.0 so that YL-01641P-$A_1$ substance was reverse-extracted into acidic water layer. After an aqueous solution is separated to remove ethylacetate, another 100 mℓ of ethylacetate was added to the solution. Then, sodium hydroxide was added under shaking to the water layer to adjust pH to 7.0 so that YL-01641P-$A_1$ substance was reverse-dissolved into ethylacetate layer. After the extractant of ethylacetate was separated from the water layer, anhydrous sodium sulfate is added for drying and then anhydrous sodium sulfate was removed by filtration. The resulting filtrate was concentrated under reduced pressure to give 62.69 mg of a red powder of crude YL-01641P-$A_1$ substance (purity : 41.9 %). To the powder thus obtained, 5 mℓ of water was added and then 1N-hydrochloric acid was added drop by drop to dissolve the powder completely. The solution was adjusted to pH 5.0 with sodium hydroxide to prepare an aqueous solution of crude YL-01641P-$A_1$ substance.

The aqueous solution of crude YL-01641P-$A_1$ substance was charged on a column (an external diameter of 1.2 cm and the length of 45 cm) packed with 30 mℓ of Diaion CHP-20P (Mitsubishi Chemical Industries, Ltd.) filled with water so as to adsorb the objective substance YL-01641P-$A_1$. After the column was washed with 50 mℓ of water, the gradient elution was effected with 0 to 80 % acetone solution (totally 600 mℓ). The eluent was divided into fractions (10 mℓ each) by a fraction collector. Among the thus eluted fractions, all fractions in which red-orange substances were eluted were examined by thin layer chromatography using silica gel plates which were developed with chloroform : methanol = 90 : 10 to confirm the presence of the objective substance YL-01641P-$A_1$ (Rf = 0.38). From this result, it was confirmed that the objective substance YL-01641P-$A_1$ was eluted in fractions 46 to 52. The purest fractions 48 to 50 were collected and concentrated under reduced pressure. After over-saturated sodium bicarbonate solution was added to adjust pH over 7.0, the extraction was carried out twice with 25 mℓ of ethylacetate. After the resulting extract was dried over anhydrous sodium sulfate and then anhydrous sodium sulfate was removed, the filtrate was concentrated and dried overnight in a desiccator in vacuo to give 25.3 mg of a red powder containing YL-01641P-$A_1$ substance (purity 86.2 %).

Example 3

A culture medium "A" (pH 7) containing 1.0 % of glucose, 2.0 % of potato starch, 0.5 % of polypeptone, 0.5 % of yeast extract and 0.4 % of calcium carbonate was prepared. The resulting culture medium was divided into a plurality of 500 mℓ Erlenmeyer flasks each containing 100 mℓ of the culture medium and then was sterilized at 120 °C for 20 minutes. Hyphae of the strain Nocardia sp. YL-01641P which had grown well on Bennett's agar medium was scratched off and inoculated to the culture medium which was then incubated under shaking at 27 °C for 96 hours to prepare seed cultures (1).

800 mℓ of the culture medium "A" was prepared and divided and poured into a plurality of 500 mℓ Erlenmeyer flasks each containing 100 mℓ of the culture medium. After they were sterilized at 120 °C for 20 minutes, the seed culture (1) was inoculated in the culture medium in a proportion of 2 % and incubated at 27 °C for 96 hours under shaking to prepare a seed culture (2).

For fermentation, 20ℓ of a production medium (pH 7.0) containing 5 % of copra meal, 2.5 % of roasted wheat germ, 0.15 % of calcium carbonate and 0.09 % of antifoaming agent NKL-5430 (Nippon Yushi Co., Ltd.) was prepared in two jar fermenters (30 ℓ jar) and was sterilized at 120 °C for 30 minutes.

400 mℓ of the seed culture (2) was inoculated to each jar fermenter. After fermentation was carried out for 3 days under stirring at 200 rpm with airation at a flow rate of 3 ℓ/min, the antibacterial activity of YL-01641P-$A_1$ substance to Bacillus subtilis ATCC 6633 became the maximum. The resulting fermentation broth was adjusted to pH of 3.5 with hydrochloric acid. Radio Light # 600 (Showa Chemical Industries, Inc.) was added, stirred and then filtered. The filtrate was adjusted to pH of 7.0 with sodium hydroxide and the precipitated insoluble substances were removed by filtration to give 27 ℓ of a filtrate of the fermentation broth. In order to extract YL-01641P-$A_1$ substance from the filtrate, 18 ℓ of ethylacetate was added and the filtrate was adjusted to pH of 7.0 with sodium hydroxide under shaking so that YL-01641P-$A_1$ substance was transferred to ethylacetate layer. The extraction was carried out twice. After a layer of the filtrate of the fermentation broth containing YL-01641P-$A_1$ substance was separated, anhydrous sodium sulfate was added for drying and then anhydrous sodium sulfate was separated by filtration. Then, the filtrate was concentrated to 2 ℓ under reduced pressure. After 1 ℓ of water was added to a condensate, a water layer was adjusted to pH of 2.0 with hydrochloric acid under shaking so that YL-01641P-$A_1$ substance was reverse-extracted into the acidic water. Then, the aqueous solution was separated to remove an ethylacetate layer. The procedure was carried out twice to obtain about 2 ℓ of an acidic solution containing YL-01641P-$A_1$ substance. Then, another 1 ℓ of ethylacetate was added and the water layer was adjusted to pH of 7.0

with sodium hydroxide. After YL-01641P-$A_1$ substance is extracted into ethylacetate layer, a water layer is removed. This procedure was carried out twice to obtain about 2 ℓ of an extract of ethylacetate containing YL-01641P-$A_1$ substance. Then, anhydrous sodium sulfate was added to the extract of ethylacetate for drying and then anhydrous sodium sulfate was separated by filtration. The filtrate was concentrated under reduced pressure to give 436 mg of a red powder containing crude YL-01641P-$A_1$ substance. 153 mg of the crude red powder containing the crude YL-01641P-$A_1$ was dissolved in 2 mℓ of methanol. After the methanol solution was divided into portions of 0.4 mℓ each, the solution is fractioned by liquid chromatography in a column (2 cm × 25 cm) of YMC-Pack R-ODS-5 (a product of Yamamura Chemical Institute) which was eluted with a mixture of 0.05 M phosphate buffer (pH 4.35) / acetonitrile / tetrahydrofuran (75 : 10 : 15) at a flow rate of 9 mℓ/min. The presence of the objective substance was detected by UV absorption at 480 nm. The substance YL-01641P-$A_1$ was eluted at a retention time of 15 min 48 sec. This fraction was collected. Purity of this fraction was confirmed by liquid chromatography [a column (6 mm × 150 mm) : YMC-Pack A-312 (S-5120-ODS) (a product of Yamamura Chemical Institute), an eluting solution : 0.05M-phosphoric acid buffer (pH 4.35) / acetonitrile / tetrahydrofuran (70:15:15), a flow rate : 1 mℓ per minute, a retention time : 5 min 51 sec]. The fraction was concentrated under reduced pressure to evaporate organic solvents. The resulting eluted liquid (about 120 mℓ) was adjusted to pH higher than 7.0 with an excessive amount of sodium hydrogen carbonate solution and then extraction was carried out with 100 mℓ of ethylacetate. The procedure was effected twice to give about 200 mℓ of ethylacetate solution containing YL-01641P-$A_1$ substance. anhydrous sodium sulfate was added to the solution for drying and then anhydrous sodium sulfate was separated by filtration. The resulting solution was concentrated under reduced pressure and then dried overnight in a desiccator in vacuo to give 83 mg of pure YL-01641P-$A_1$ substance as a red powder. To 40.0 mg of the red powder, 1 mℓ of water was added and then 0.5 % aqueous solution of tartaric acid was added drop by drop under stirring until the red powder was completely dissolved. When the substance YL-01641P-$A_1$ was completely dissolved, the pH of the solution was 4.53. The resulting aqueous solution of tartaric acid containing YL-01641P-$A_1$ substance was lyophilized to give 42.6 mg of a crystalline powder of tartrate of pure YL-01641P-$A_1$ substance. This tartrate have a remarkably higher solubility in water than that of the free base of YL-01641P-$A_1$ substance.

Example 4

Preparation of YL-01641P-$A_2$, -$B_2$ and -$B_1$ substances

[A] A culture medium "A" (pH 7) containing 1.0 % of glucose, 2.0 % of potato starch, 0.5 % of polypeptone, 0.5 % of yeast extract and 0.4 % of calcium carbonate was prepared. The resulting culture medium was divided into a plurality of 500 mℓ Erlenmeyer flasks each containing 100 mℓ of the culture medium and then was sterilized at 120 °C for 20 minutes. Hyphae of a strain Nocardia sp. YL-01641P which had grown well on Bennett's agar medium was inoculated in the seed medium which was then incubated under shaking at 27 °C for 96 hours to prepare seed culture (1). Another 800 mℓ of the culture medium "A" was prepared and divided into a plurality of 500 mℓ Erlenmeyer flasks each containing 100 mℓ of the culture medium. After the culture medium were sterilized at 120 °C for 20 minutes, the seed culture was inoculated in the seed culture (1) in a proportion of 2 % and incubated at 27 °C for 96 hours under shaking to prepare a seed culture (2). For mass fermentation, 20ℓ of a production medium (pH 7.0) containing 5 % of copra meal, 2.5 % of wheat gem, 0.15 % of calcium carbonate and 0.09 % of anti-foam agent NKL-5430 (Nippon Yushi Co., Ltd.) was prepared in two jar fermenters (30 ℓ jar) and was sterilized at 120 °C for 30 minutes. 400 mℓ of the seed culture (2) was inoculated to each jar fermenter. Fermentation was carried out for 2 days under stirring at 200 rpm with airation at a flow rate of 30 ℓ/min.

[B] After 19 ℓ of the fermentation broth thus obtained was adjusted to pH of 3.5 with hydrochloric acid, Radio Light # 600 (Showa Chemical Industries, Inc.) was added, stirred and then filtered to give 15 ℓ of filtrate. In order to extract the objective substances from the filtrate, 10 ℓ of ethylacetate was added and the pH was adjusted to 7.0 with sodium hydroxide with shaking so that the objective substances was extracted in an ethylacetate layer. The extraction was carried out twice. After the extract containing the objective substances was separated from ethylacetate, anhydrous sodium sulfate was added for drying and then anhydrous sodium sulfate was removed by filtration. Then, the resulting extract was concentrated to 2 ℓ under reduced pressure. To the resulting concentrated extract, 1 ℓ of water was added and then the water layer was adjusted to pH of 2.0 with hydrochloric acid so that the objective substances was reverse-extracted into acidic water. Then, the aqueous solution was separated to remove ethylacetate layer. This procedure was carried out twice to obtain about 2 ℓ of an acidic solution containing the objective substances. Then, another 1 ℓ of ethylacetate was added and the water layer was adjusted to pH of 7.0 with sodium hydroxide with shaking so that the objective substance was extracted in ethylacetate layer. Then, a water layer was separated. This procedure was carried out twice to obtain about 2 ℓ of an extract of ethylacetate containing the objective substances. Then, anhydrous sodium sulfate was added to the ethylacetate extract for drying and then anhydrous sodium sulfate was removed by filtration. The resulting filtrate was concentrated to dryness under reduced pressure to give 302 mg of crude product containing the objective substances.

[C] 302 mg of the crude product was dissolved in 5 mℓ of methanol. The methanol solution was divided into portions each has a volume of 0.5 mℓ. The portions were examined by liquid chromatography to separate three objective substances according to difference in retention times under the following conditions : a column (2 cm × 25 cm) of YMC-Pack R-ODS-5 (a product of Shimazu Techno-Research) which was eluted with 0.02 M-phosphate buffer (pH 4.2) / acetonitrile / tetrahydrofuran / methanol (67 : 15 : 8 : 10) at a flow rate of 7 mℓ/min. Detection was effected by UV absorption at 254 nm. The presence of the objective substances was effected by thin layer chromatography of silica gel plates (Kieser Gel $60F_{254}$, 20 cm × 20 cm, a product of Merk) which were developed with chloroform / ethylacetate / methanol / aqueous ammonia = 70:30:10:0.1. Purity of the objective substances was also confirmed by liquid chromatography for analysis [a column (STR-ODS-H, 4.6 mm × 250 mm, a product of Shimazu Techno-Research) was eluted with an eluent of 0.02 M-phosphoric acid buffer (pH 4.2) / acetonitrile / tetrahydrofuran / methanol (= 65:15:10:10) at a flow rate of 0.8 mℓ/min].

(1) An objective substance obtained from a fraction eluted at a retention time of 14 min 48 sec in the liquid chromatography.

This substance is a single substance having Rf value of 0.17 in the thin layer chromatography and a retention time of 5 min 31 sec in the liquid analytical chromatography.

After organic solvents in this fraction was removed under reduced pressure, sodium hydrogen carbonate was added to adjust the pH higher than 7.0 and then the extraction was carried out with ethylacetate. anhydrous sodium sulfate was added for drying and then anhydrous sodium sulfate was removed by filtration. The filtrate was concentrated to dryness under reduced pressure and dried overnight in a desiccator in vacuo to obtain a red powder (8.0 mg) possessing the following physicochemical properties:

(1) Appearance: Red needle-like crystal

(2) Nature: Basic

(3) Solubility in solvents: soluble in methanol, ethanol, acetone, ethylacetate, benzene, toluene and chloroform, but sparingly soluble in water and substantially insoluble in hexane.

(4) Ultraviolet Absorption Spectrum: An ultraviolet absorption spectrum of the compound YL-01641P-$A_2$ measured in methanol is shown in Figure 4.

(5) Mass Spectrum (FAB): 748 (M + 1)

(6) Molecular weight: 747.792

(7) Molecular formula: $C_{37}H_{49}NO_{15}$

(8) $^1$H-NMR Spectrum: $^1$H-NMR Spectrum (500 MHz, $CDCl_3$) of the compound YL-01641P-$A_2$ is shown in Figure 5.

From the physicochemical properties, this substance is concluded to be YL-01641P-$A_2$ substance represented by the following formula:

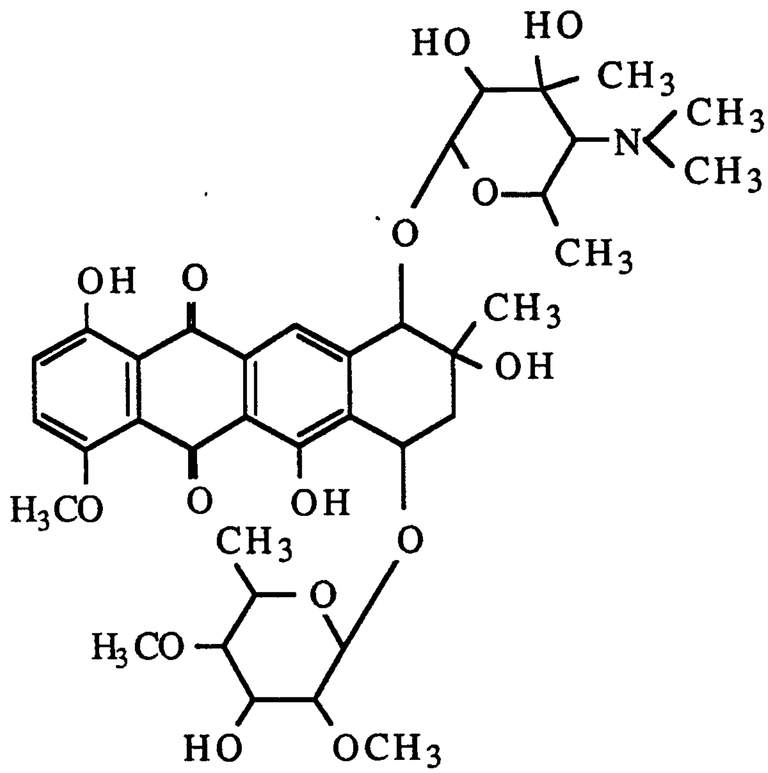

(2) An objective substance obtained from a fraction eluted at a retention time of 31 min 30 sec in the liquid chromatography.

This substance is a single substance having Rf value of 0.15 in the thin layer chromatography and a retention time of 10 min 14 sec in the liquid chromatography for analysis. This fraction was extracted, concentrated and dried according to the same method as mentioned above to obtain a dark-red powder (6.7 mg) possessing the following physicochemical properties:

(1) Appearance: Dark-red needle-like crystal

(2) Nature: Basic

(3) Solubility in solvents: soluble in methanol, ethanol, acetone, ethylacetate, benzene, toluene and chloroform, but sparingly soluble in water and insoluble in hexane.

(4) Ultraviolet Absorption Spectrum: An ultraviolet absorption spectrum of the substance YL-01641P-$B_2$ measured in methanol is shown in Figure 6.

(5) Mass Spectrum (FAB): 734 (M + 1)

(6) Molecular weight: 733.765

(7) Molecular formula: $C_{36}H_{47}NO_{15}$

(8) $^1$H-NMR Spectrum: $^1$H-NMR Spectrum (500 MHz, $CDCl_3$) of the substance YL-01641P-$B_2$ is shown in Figure 7.

From the physicochemical properties, this substance is concluded to be YL-01641P-$B_2$ substance represented by the following formula:

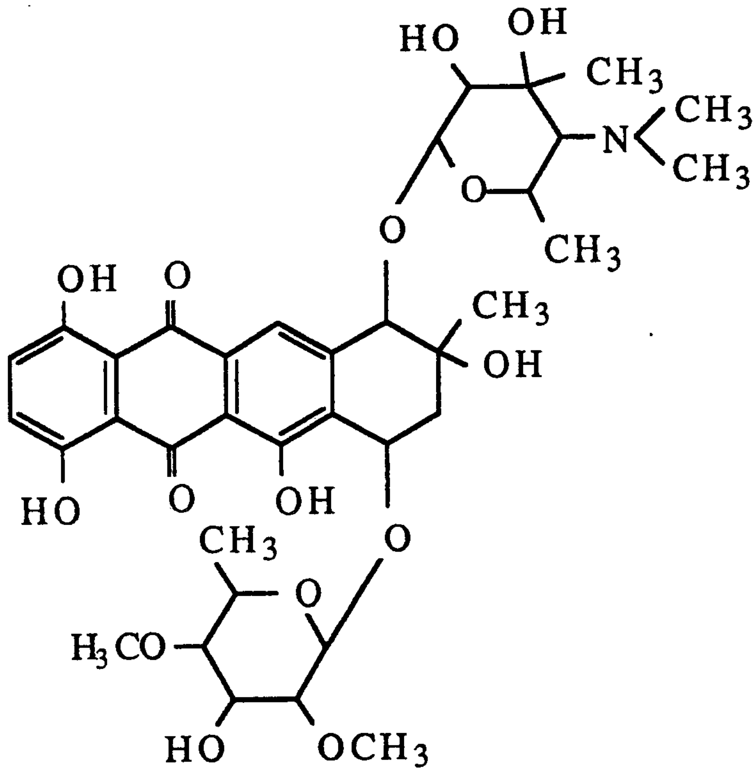

(3) An objective substance obtained from a fraction eluted at a retention time of 47 min 45 sec in the liquid chromatography

This substance is a single substance having Rf value of 0.28 in the thin layer chromatography and a retention time of 16 min 08 sec in the liquid chromatography for analysis. This fraction was extracted, concentrated and dried according to the same method as mentioned above to obtain a dark-red powder (69.8 mg) possessing the following physicochemical properties:

(1) Appearance: Dark-red needle-like crystal

(2) Nature: Basic

(3) Solubility in solvents: soluble in methanol, ethanol, acetone, ethylacetate, benzene, toluene and chloroform, but difficult to be dissolved in water and insoluble in hexane.

(4) Ultraviolet Absorption Spectrum: An ultraviolet absorption spectrum of the substance YL-01641P-$B_1$ measured in methanol is shown in Figure 8.

(5) Mass Spectrum (FAB): 776 (M + 1)

(6) Molecular weight: 775.802

(7) Molecular formula: $C_{38}H_{49}NO_{16}$

(8) $^1$H-NMR Spectrum: $^1$H-NMR Spectrum (500 MHz, $CDCl_3$) of the substance YL-01641P-$B_1$ is shown in Figure 9.

(9) $^{13}$C-NMR Spectrum: $^{13}$C-NMR Spectrum of the substance YL-01641P-$B_1$ (125 MHz, $CDCl_3$) is shown in Figure 10.

From the physicochemical properties, the present substance is concluded to be YL-01641P-$B_1$ substance represented by the following formula:

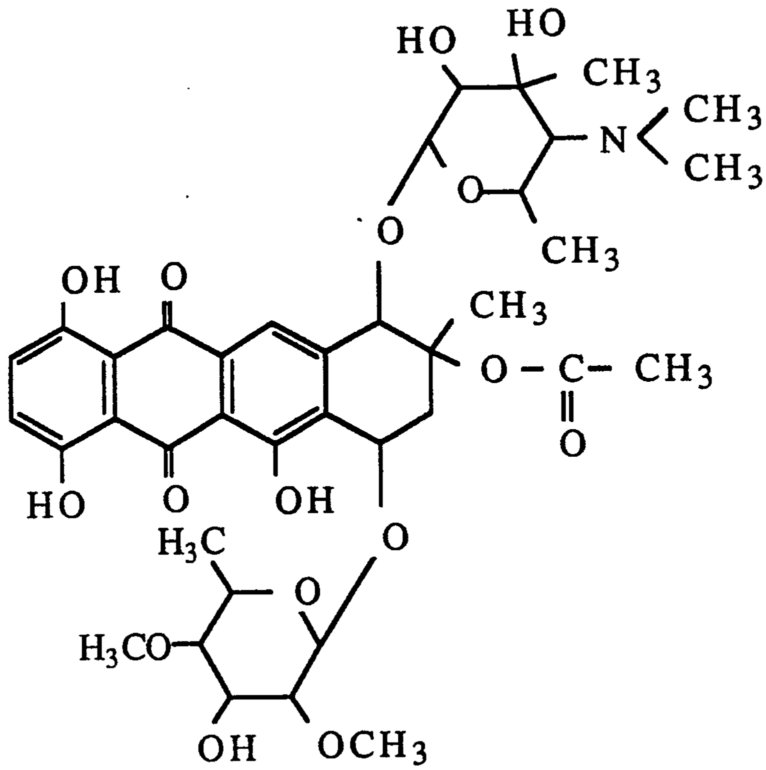

[D] After a proper amount of water was added to each 3 mg of YL-01641P-$A_2$ substances, 3 mg of YL-01641P-$B_2$ substances and to 30 mg of YL-01641P-$B_1$ substances respectively, 0.5 % aqueous solution of tartaric acid was added drop-wise until each powder dissolved completely. When these compounds were dissolved completely, the pH values were found to be in a range from 4.5 to 4.6. The aqueous solutions of tartaric acid containing the compounds were lyophilized to give 3.17 mg of tartrate of pure YL-01641P-$A_2$, 3.17 mg of tartrate of YL-01641P-$B_2$ and 31.68 mg of tartrate of pure YL-01641P-$B_1$, respectively. These tartrates show the solubilities in water much higher than that of the free base of the compounds which are starting materials.

Example 5

## Preparation of YL-01641P-$C_1$ substance

The seed cultures (1) and (2) were prepared by the same process as Example 3. Then, 400 mℓ of the seed culture (2) was inoculated in the same seed medium which had been prepared in a jar fermenter (30 ℓ jar) by the same process as Example 3. After sterilization, the inoculated seed culture (3) was incubated for 72 hours under stirring at 200 rpm with airation at a flow rate of 30 ℓ/min.

For fermentation, 230 ℓ of the same fermentation medium as Example 3 was prepared and sterilized at 121 °C for 45 minutes and was inoculated with 4 ℓ of the seed culture (3). After fermentation which was carried out for three days under stirring at 75 rpm with airation at a flow rate of 150 ℓ/min, YL-01641P-$C_1$ substance showed the maximum antibacterial activity to Bacillus subtilis ATCC 6633.

After the fermentation broth thus obtained was adjusted to pH of 2.0 with hydrochloric acid, Radio Light # 600 (Product of Showa Chemical Industries, Inc) was added and stirred. After one hour, the fermentation broth was filtered. The resulting filtrate was adjusted to pH 7.0 with 1N-sodium hydroxide. After precipitates were separated, 205 ℓ of a clear filtrate was obtained.

In order to extract YL-01641P-$C_1$ substance from the clear filtrate, the filtrate was applied to a column (30 cm × 150 cm) packed with 20 ℓ of Diaion HP-20 (Mitsubishi Chemical Industries, Inc) at a rate of SV = 2.5 so as to adsorb YL-01641P-$C_1$ substance. After washing with water, 4 ℓ of 30 % aqueous acetone solution, 60 ℓ of 60 % aqueous acetone solution and 60 ℓ of 60 % acetone 1 / 0.05 M phosphate buffer

were flowed successively to elute the objective substance YL-01641P-$C_1$.

Detection of the objective substance was effected by thin layer chromatography [Kieser Gel $60F_{254}$, 20 cm × 20 cm (a product of Merk), chloroform / ethyl acetate / methanol / 28 % aqueous ammonia (=70:30:10:0.1), Rf value of the objective substance YL-01641P-$C_1$ : 0.3]. The objective substance YL-01641P-$C_1$ was contained in such fractions (80 ℓ) that were eluted after the elution with 60 % aqueous acetone solution. After the eluent was concentrated to about 30 ℓ to remove acetone, 20 ℓ of ethylacetate was added and sodium hydroxide was added under shaking so that the pH of the water layer was adjusted to 7.0 in order to extract the objective substance YL-01641P-$C_1$ into an ethylacetate layer. The extraction was carried out twice. After the resulting extract was concentrated under reduced pressure to about 3 ℓ, 2 ℓ of water was added and then hydrochloric acid was added under shaking to adjust the pH of a water layer to 2.0 so that the objective substance was reverse-extracted into acidic water. This procedure was carried out twice to give 4 ℓ of an acidic solution containing the objective substance. Then, another 2 ℓ of ethylacetate was added and then sodium hydroxide was added under shaking to adjust the pH to 7.2 so that the objective substance YL-01641P-$C_1$ was extracted in an ethylacetate layer. This procedure was carried out twice to give 4 ℓ of an extract containing the objective substance. In order to confirm the presence of the objective substance, the same thin layer chromatography as above was used at every extraction stage. The ethylacetate solution containing the objective substance YL-01641P-$C_1$ was dried over anhydrous sodium sulfate, concentrated at 40 °C under reduced pressure and dried overnight in a desiccator in vacuo to give 8.8 g of a crude powder containing YL-01641P-$C_1$. To the crude powder, 50 mℓ of water was added and then hydrochloric acid was added until the powder dissolved completely to obtain an aqueous solution containing the objective substance YL-01641P-$C_1$. This solution was charged onto a column (4 cm x 115 cm) packed with 1 ℓ of Diaion HP-20 (Mitsubishi Chemical Industries, Inc.) which had been washed with water. After adsorption, the column was washed with water and then the gradient elution was effected with 30 to 60 % aqueous acetone solutions (totally 8 ℓ). Eluent was collected as fractions each containing 16 g of the eluent by a fraction collector. Detection of the objective substance in the eluent was effected by the same thin layer chromatography as above and found that the objective substance was contained in fraction No. 341 to 385. These fractions were collected and concentrated at 40 °C under reduced pressure to eliminate acetone. After the same amount of ethylacetate as the resulting concentrated solution was added, the pH of a water layer was adjusted to 7.2 and the objective substance was extracted. This procedure was carried out twice. This extract was dried over anhydrous sodium sulfate, concentrated at 40 °C under reduced pressure and dried overnight in a desiccator in vacuo to give 3.0 g of a crude powder containing the objective substance YL-01641P-$C_1$. To 1.0 g of the crude powder, 2 mℓ of water was added and 1N-hydrochloric acid was added drop-wise to dissolve the crude powder completely. Each 100 μℓ of the resulting solution was fractionated and collected by liquid chromatography under the following conditions : a column (2 cm x 25 cm) of STR-PREP ODS-H (a product of Shimazu Techno-Research) which was eluted with an eluent consisting of 0.02 M-phosphoric acid buffer (pH 4.2) / acetonitrile / methanol (75:15:10) at a flow rate of 8 mℓ/min. Detection was effected by UV absorption at 254 nm. A fraction containing the objective substance YL-01641P-$C_1$ eluted at a retention time of 21 min 6 sec was collected and concentrated under reduced pressure at 40 °C to remove organic solvents. After the same amount of ethylacetate as the resulting concentrated fraction was added, sodium hydrogen carbonate solution was added under shaking to adjust the pH to higher than 7.5 so that the objective substance YL-01641P-$C_1$ was extracted into an ethylacetate layer. The extraction was carried out twice. The extract was dried over anhydrous sodium sulfate, concentrated under reduced pressure at 40 °C and then dried overnight in a desiccator in vacuo to give 78 mg of a crude powder containing the objective substance YL-01641P-$C_1$. Since this powder contained a small amount of components other than the objective substance, purification was effected by the same method as above. Namely, a solution of hydrochloric acid salt was prepared by adding 1 g of water. Each 50 μℓ of this solution was fractionated and collected by liquid chromatography at a peak of the objective substance under the following conditions : a column of STR-PREP ODS-H (2 cm × 25 cm) (a product of Shimazu Techno-Research) was eluted with an eluent consisting of 0.02 M-phosphoric acid buffer (pH 4.2) / acetonitrile / methanol (55:15:30) at a flow rate of 7 mℓ/min. Detection was effected with UV absorption at 254 nm. Under this condition, a pure fraction of the objective substance YL-01641P-$C_1$ eluted at a retention time of 47 min 34 sec was collected. The presence of the objective compound YL-01641P-$C_1$ was confirmed by the same thin layer chromatography and liquid chromatography for analysis as above. In the liquid chromatography for analysis, a column of STR-PREP ODS-H (4.6 mm × 25 cm) (a product of Shimazu Techno-Research) was eluted with an eluent consisting of 0.02 M-phosphoric acid buffer (pH 4.2) / acetonitrile / methanol (55 : 15 : 30) at a flow rate of 0.8 mℓ/min. Detection was effected with UV absorption at 245 nm. The resulting fraction was concentrated under reduced pressure at 40 °C to remove organic solvents. After a same amount of ethylacetate as the concentrated fraction was added,

sodium hydrogen carbonate solution was added under shaking to adjust the pH of a water layer higher than 7.5 so that the objective substance YL-01641P-$C_1$ was extracted into an ethyl acetate layer. The extraction was carried out twice. The resulting extract was dried over anhydrous sodium sulfate, concentrated under reduced pressure at 40 °C and dried overnight in a desiccator in vacuo to give 27.5 mg of a pure powder the objective substance YL-01641P-$C_1$.

An objective substance obtained from a fraction eluted at a retention time of 47 min 34 sec in the liquid chromatography.

This substance is a single substance having Rf value of 0.28 by the thin layer chromatography and a retention time of 23 min 33 sec by the liquid analytical chromatography and possesses the following physicochemical properties:

(1) Color and shape: Orange-yellow crystalline powder

(2) Acidity: Basic

(3) Solubility in solvents: soluble in methanol, ethanol, acetone, ethylacetate, benzene, toluene and chloroform but difficult to be dissolved in water and insoluble in hexane.

(4) Ultraviolet Absorption Spectrum: an ultraviolet absorption spectrum of the compound YL-01641P-$C_1$ measured in methanol is shown in Figure 11.

(5) Mass Spectrum (FAB): 774 (M + 1)

(6) Molecular formula: $C_{39}H_{51}NO_{15}$
molecular weight : 773.830

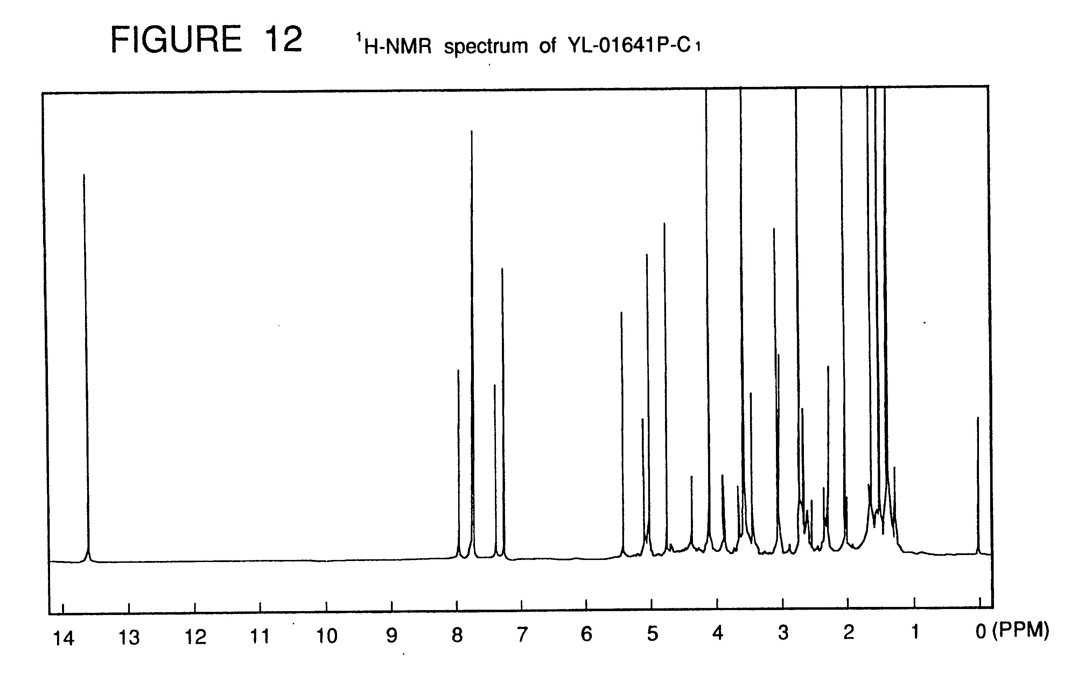

(7) $^1$H-NMR Spectrum: $^1$H-NMR Spectrum (500 MHz, $CDCl_3$) of the compound YL-01641P-$C_1$ is shown in Figure 12.

From the above physicochemical properties, this substance is concluded to be YL-01641P-$C_1$ substance represented by the following formula:

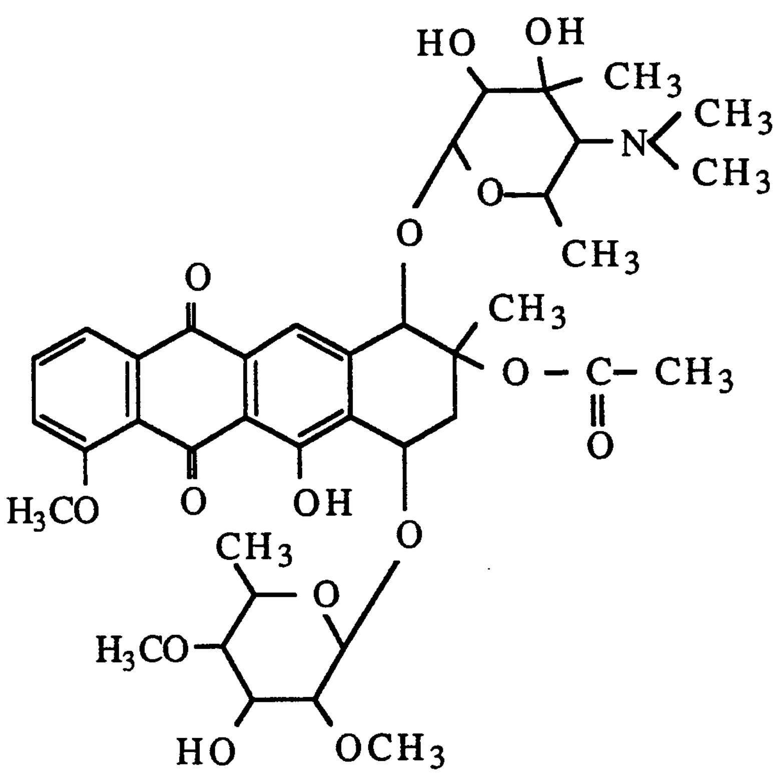

The present invention also provides the novel and useful microorganism as described above.

The novel anthracycline antitumor and antioncotic substances according to the present invention possess a cytotoxicity to various tumor cells. Now, the test results which show the cytotoxicity are described.

## (1) Cytotoxicity on tumor cells in vitro

### Method

To each 1 mℓ of L 1210* and P 388** culture medium in a concentration of 1 x $10^5$ cells/mℓ, 4 μℓ of each solution of the tartrates of the anthracycline compounds according to the present invention which was dissolved in saline in different concentrations was added respectively and incubated at 37 °C for 3 days in a $CO_2$ incubator. Then, numbers of surviving cells were counted by trypan blue exclusion. The cytotoxic activity was determined by calculating the cell growth inhibitory ratio of the numbers of surviving cells to numbers of cells of a control to which no compound was added in the different concentrations and by plotting them on a graph.

Note : Medium used:
* : RPMI-1640 + 10 % new born calf serum
**: RPMI-1640 + 5 % fetus calf serum + 5 μM 2-hydroxyethyldisulfide

### Results

| Cytotoxic activity | | | |
|---|---|---|---|
| Compound tested | $IC_{50}$ (μg/mℓ) | | |
| | L1210 | P388 | P388 adriamycin resistant cell |
| YL-01641P-$A_1$ | 0.096 | 0.032 | 0.069 |
| adriamycin | 0.087 | 0.030 | 1.1 |
| YL-01641P-$B_1$ | 0.180 | 0.180 | 0.140 |
| YL-01641P-$B_2$ | 0.110 | 0.100 | 0.130 |
| YL-01641P-$A_2$ | 0.330 | 0.160 | 0.260 |
| adriamycin | 0.090 | 0.023 | 0.320 |
| YL-01641P-$C_1$ | — | 0.180 | 0.330 |

## (2) Antitumor activity to P388 on animal

### Test Method

5 x $10^5$ cells of P-388 tumor cells were intraperitoneally inoculated to 5-weeks BDFI/SLC male mice. From the day after inoculation, tartrate of YL-01641P-$A_1$ was intraperitoneally administered for 5 days. Antitumor effect was evaluated by prolongation of life calculated by a ratio of the median survival days of a group which was administered with YL-0164P-$A_1$ to the median survival days of a control group which was not administered the same.

### Results

| Antitumor activity | | | | |
|---|---|---|---|---|
| Compound tested | Amount of administration (mg/kg) | Median survival days (day) | T/C (%) | Survival rate (after 22 days) |
| YL-01641P-$A_1$ | 1.125 × 5 | 19.0 | 146 | 1 - 8 |
| | 2.25 × 5 | 22.0 | 169 | 3 - 8 |
| | 4.5 × 5 | 10.5 | 81 | 1 - 8 |
| | 9.0 × 5 | 8.0 | 62 | 0 - 8 |
| mitomycin C | 0.50 × 5 | 22.0 | 169 | 2 - 8 |
| | 1.0 × 5 | > 22.0 | > 169 | 8 - 8 |
| control | — | 13.0 | 100 | 1-16 |

The compounds according to the present invention are administered usually by non-peroral route such as injection but can be administered also by peroral route. In the later case, the compound according to the present invention is blended or mixed with pharmaceutically acceptable solid or liquid carrier to be formed in powder, granule, tablet, syrup etc.

In general administration method to animals, the compound according to the present invention is administered by injection intraperitoneally, intravascularly per vein or per arteria or by local injection. The compound according to the present invention can be administered continuously or discontinuously on condition that the total dose do not exceed a predetermined amount which depends on the results of animal tests and the other factors. The dose can be modified of course in accordance with administration routes, conditions of patients or animals to be treated including age, weight, sex, sensibility, diet, administration time and administration method, agents used together and patient's symptom. Usual dose of the compound according to the present invention can be in a range from 0.3 mg/kg to 15 mg/kg a day a dose when the compound is used as antitumor agent. The amount and times of administration must be determined by the sufficient-quantity-determining test carried out by medical specialists on the basis of the above-mentioned guideline. These conditions are considered also for peroral administration.

**Claims**

1. Anthracycline substances (I) represented by the following plane chemical formula:

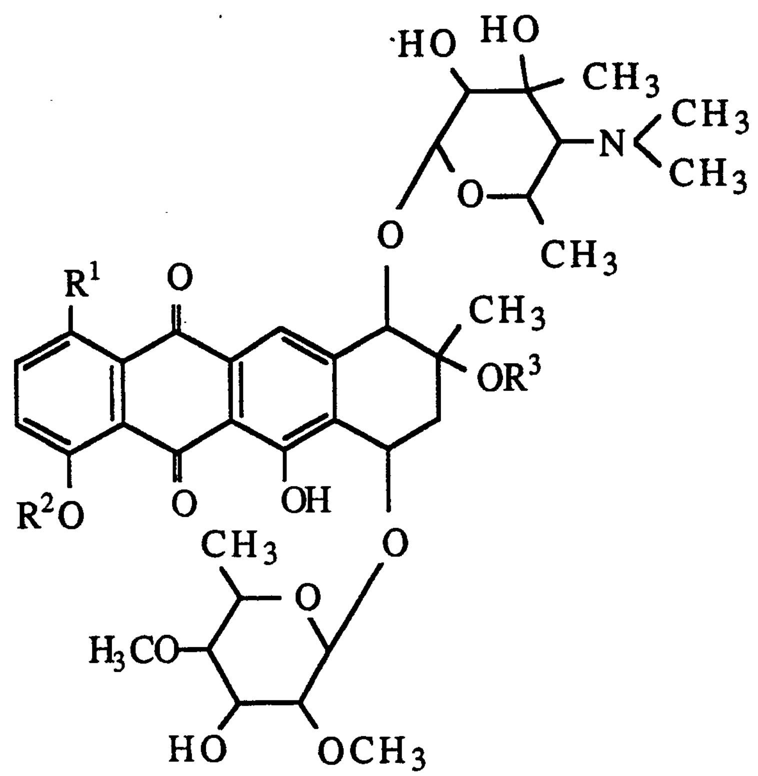

(I)

wherein $R^1$ represents a hydrogen atom or a hydroxyl group, $R^2$ represents a hydrogen atom or a lower alkyl group and $R^3$ represents a hydrogen atom or a lower alkanoyl group
or acid addition salts thereof.

2. The anthracycline substances (I) or acid addition salts thereof set forth in Claim 1 wherein $R^1$ is a hydrogen atom or a hydroxyl group, $R^2$ is a hydrogen atom or a methyl group and $R^3$ is a hydrogen atom or an acetyl group.

3. The anthracycline substances (I) or acid addition salts thereof set forth in Claim 2 wherein $R^1$ is a hydroxyl group, $R^2$ is a methyl group and $R^3$ is an acetyl group.

4. A microorganism classified in genus Nocardia and having an ability to produce the anthracycline substances set forth in Claim 1.

5. The microorganism set forth in Claim 4 wherein the microorganism belongs to genus Nocardia is Nocardia sp. YL-01641P (FERM BP-2737) or a mutant thereof.

6. A process for producing an anthracycline substance characterized by the steps of incubating a microorganism classified in genus Nocardia and having an ability to produce the anthracycline substance in a culture medium so as to produce and store the anthracycline substance in a fermentation broth and collecting the produced and stored the anthracycline substance from the fermentation broth.

7. The process set forth in Claim 6 wherein the microorganism classified in genus Nocardia is a strain Nocardia sp -YL01641P (FERM BP-2737) or a mutant thereof.

Claims for the following Contracting States : ES, GR

1. A process for producing anthracycline substances (I) represented by the following plane chemical formula:

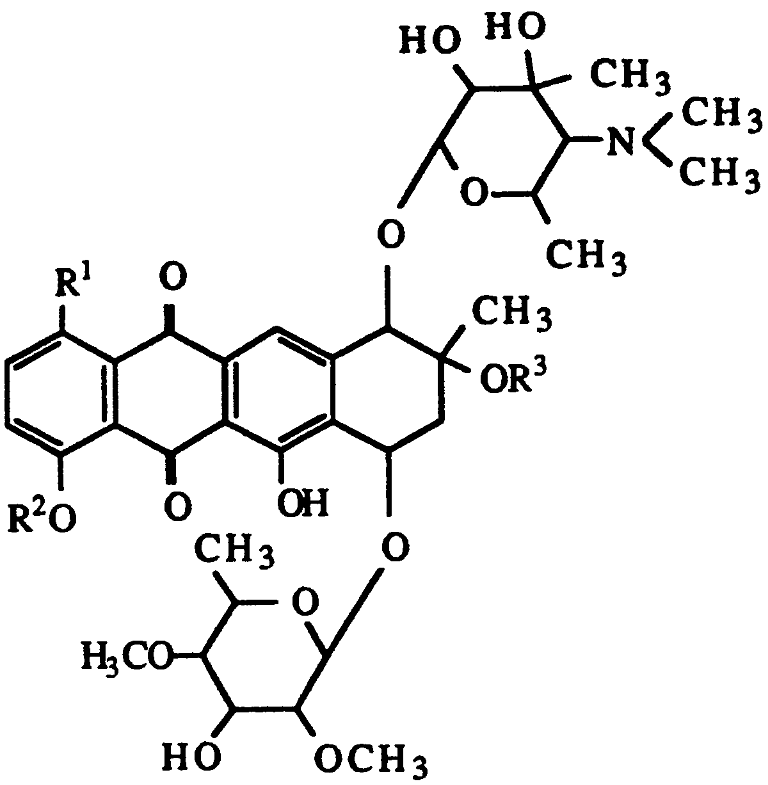

(I)

wherein $R^1$ represents a hydrogen atom or a hydroxyl group, $R^2$ represents a hydrogen atom or a lower alkyl group and $R^3$ represents a hydrogen atom or a lower alkanoyl group
or acid addition salts thereof, characterized by the steps of incubating a microorganism classified in genus Nocardia and having an ability to produce the anthracycline substances in a culture medium so as to produce and store the anthracycline substances in a fermentation broth and collecting the produced and stored the anthracycline substances from the fermentation broth.

2. The process set forth in Claim 1 wherein $R^1$ is a hydrogen atom or a hydroxyl group, $R^2$ is a hydrogen atom or a methyl group and $R^3$ is a hydrogen atom or an acetly group.

3. The process set forth in Claim 2 wherein $R^1$ is a hydroxyl group, $R^2$is a methyl group and $R^3$ is an acetly group.

4. The process set forth in Claim 1 wherein the microorganism classified in genus Nocardia is a strain Nocardia sp. YL-01641P (TERM BP-2737) or a mutant thereof.

5. A microorganism classified in genus Nocardia and having an ability to produce the anthracycline substances in the process set forth in Claim 1.

6. The microorganism set forth in Claim 4 wherein the microorganism belongs to genus Nocardia is Nocardia sp. YL-01641P (TERM BP-2737) or a mutant thereof.

FIGURE 1

Ultraviolet absorption of YL-01641-$A_1$

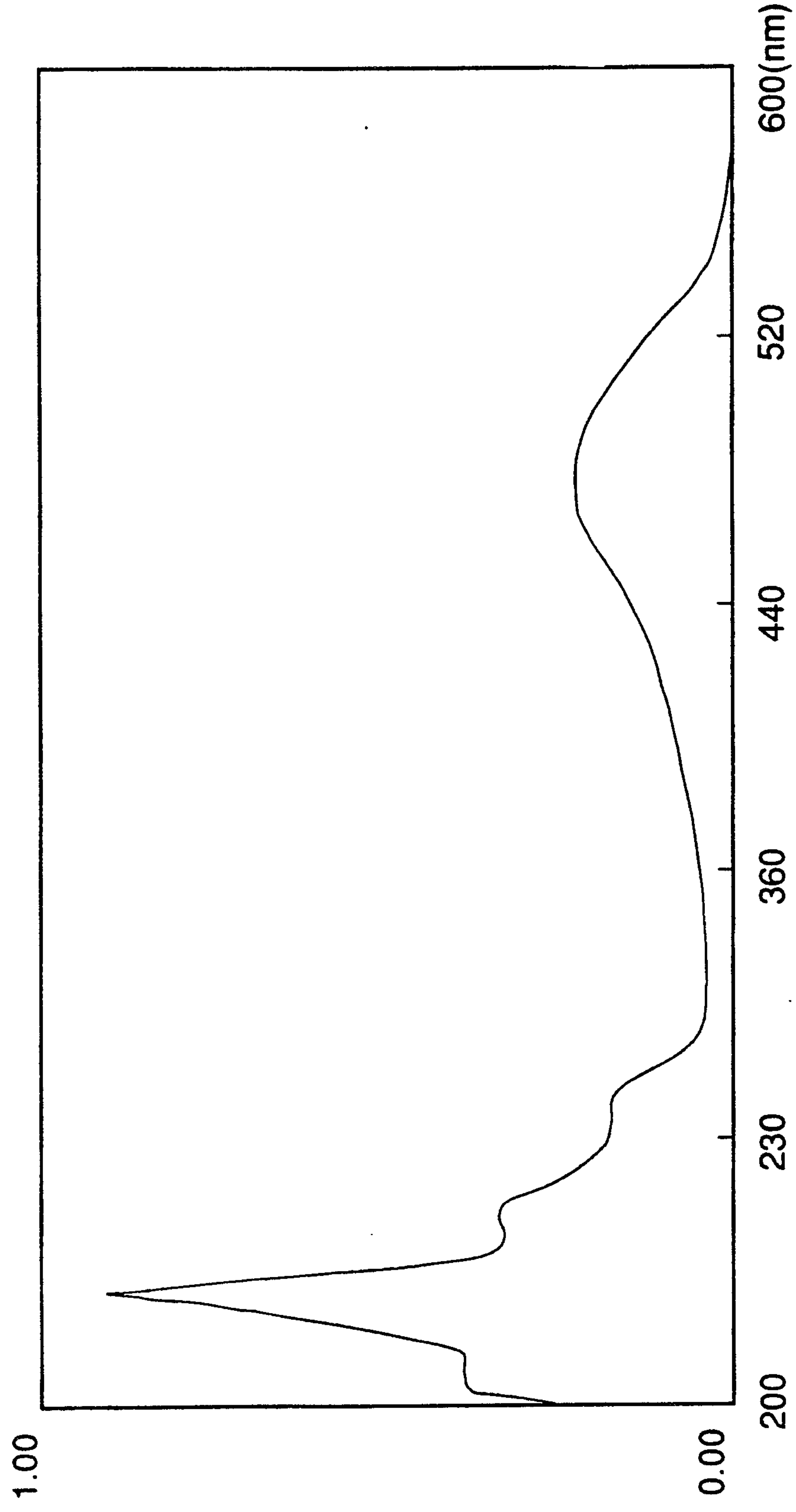

FIGURE 2 $^1$H-NMR spectrum of YL-01641P-$A_1$

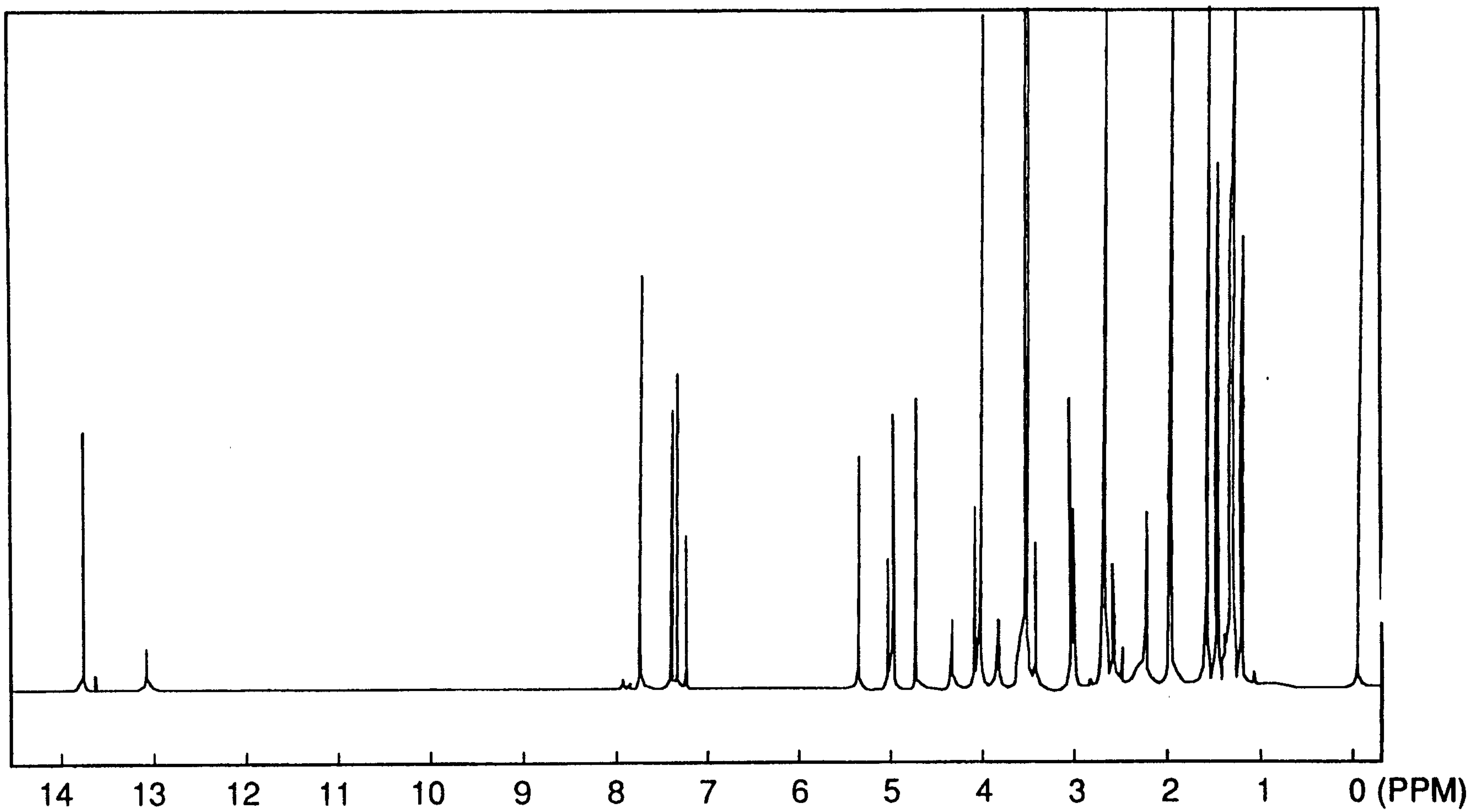

# FIGURE 3

$^{13}C$-NMR spectrum of YL-01641P-$A_1$

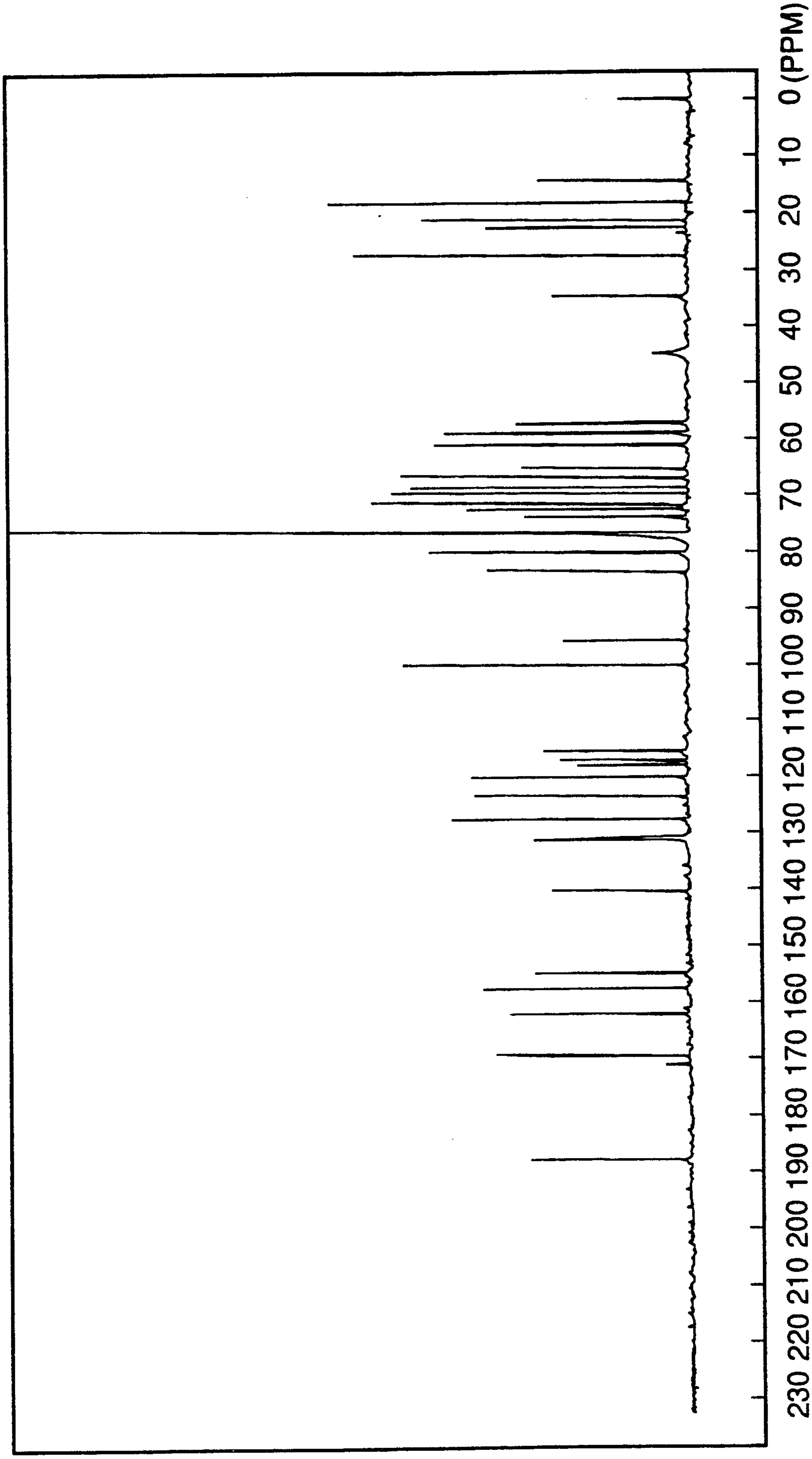

FIGURE 4

Ultraviolet absorption spectrum of YL-01641P-A 2

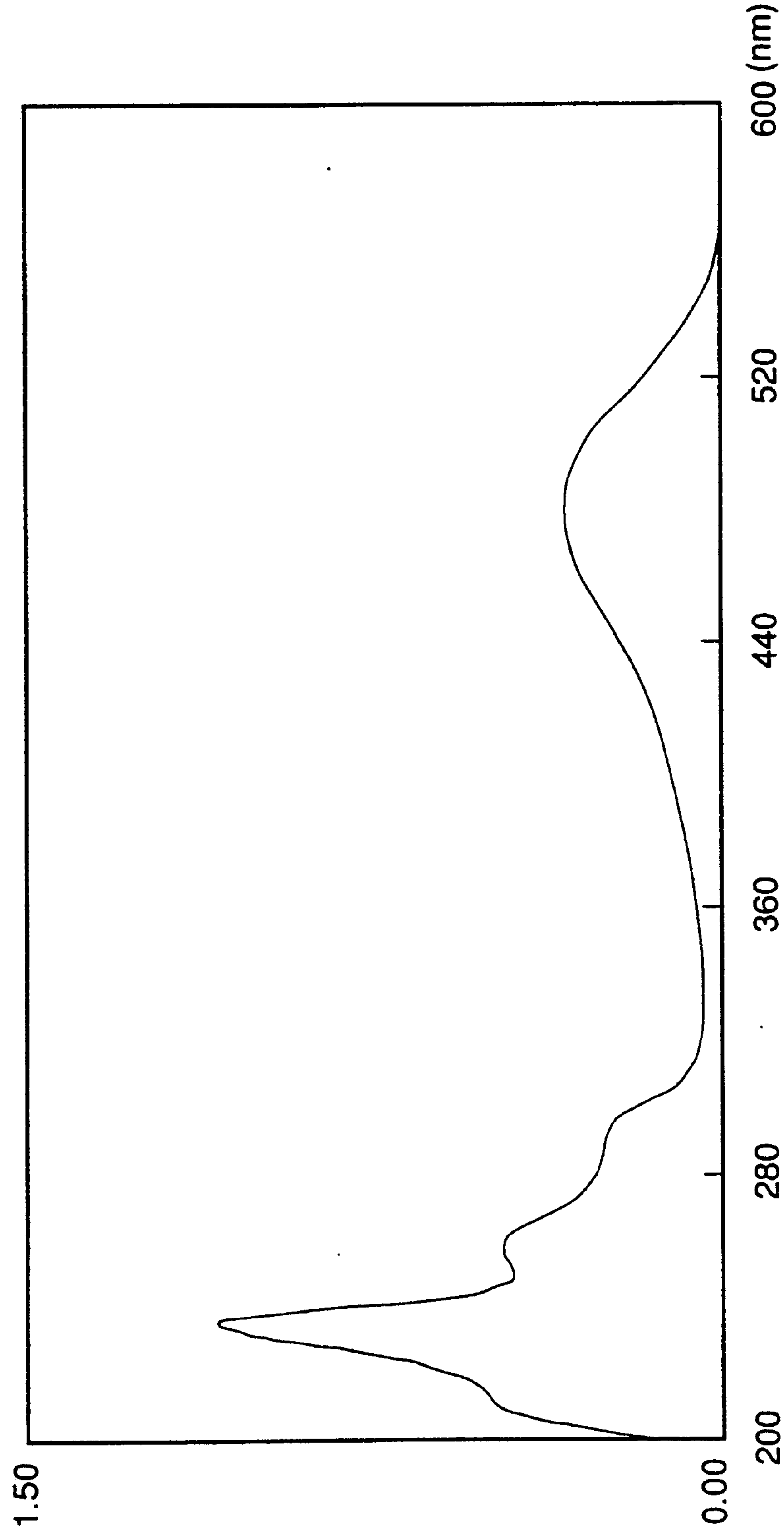

FIGURE 5 $^1$H-NMR spectrum of YL-01641P-A$_2$

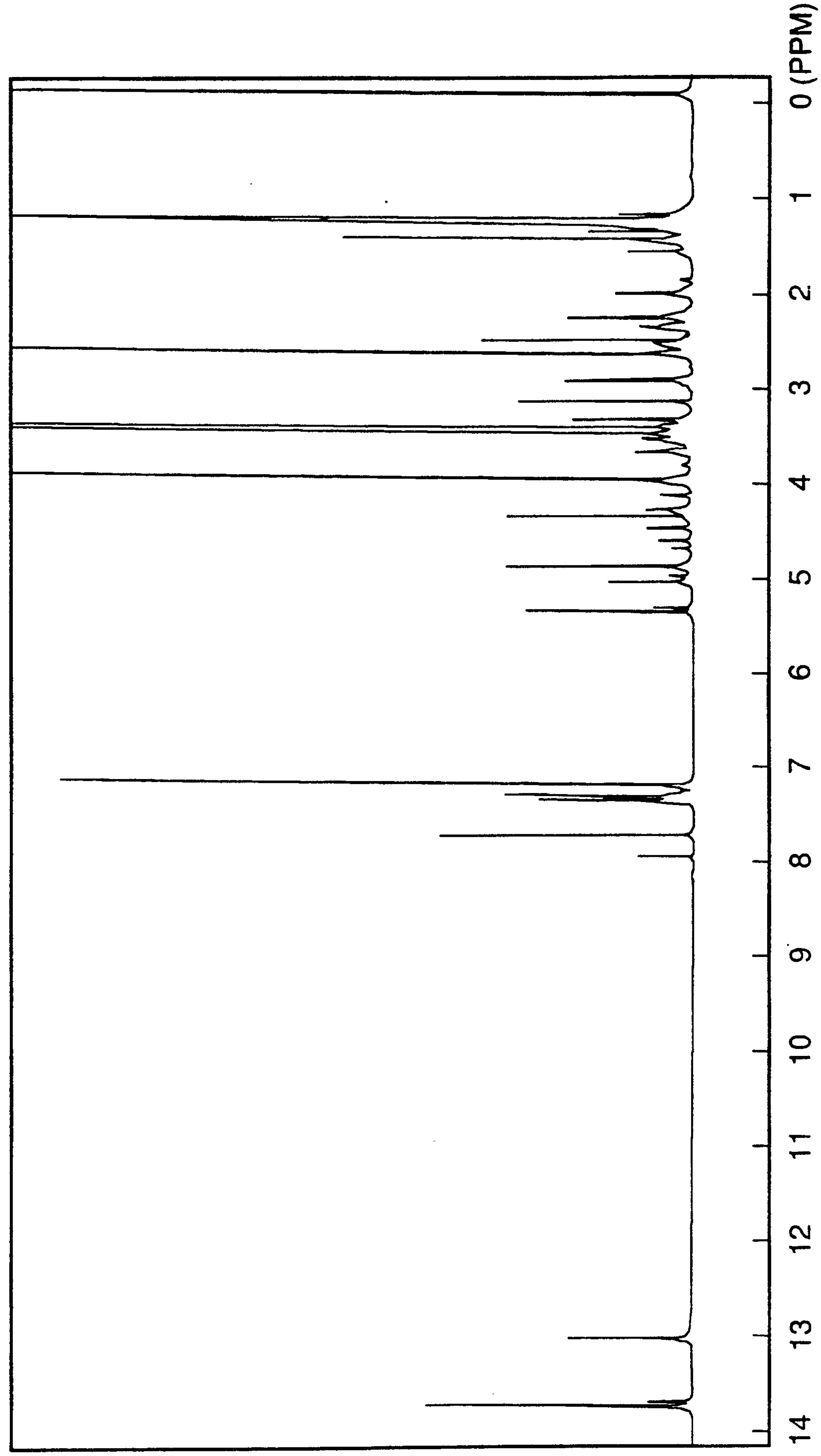

# FIGURE 6

Ultraviolet absorption spectrum of YL-01641P-B 2

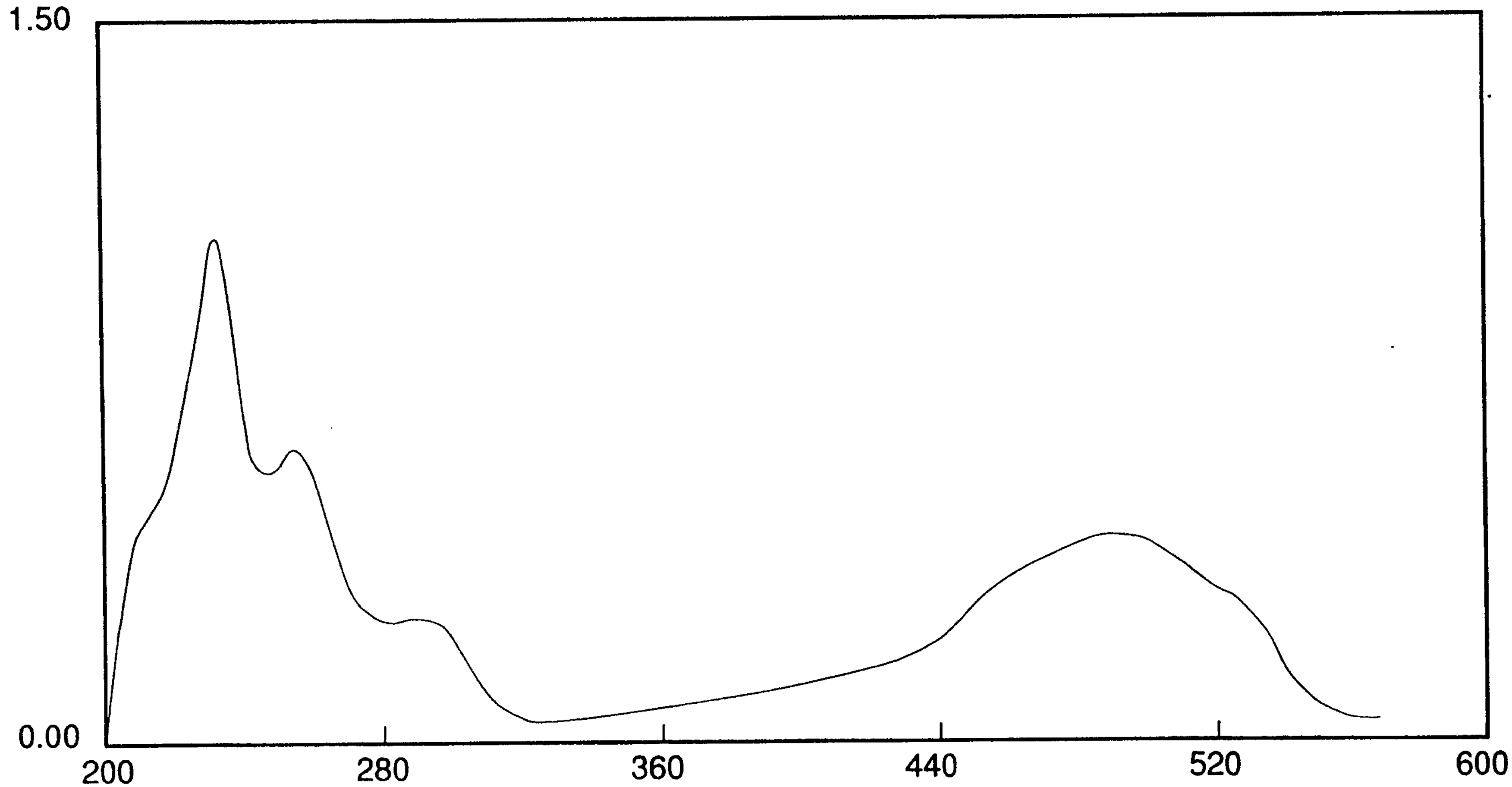

FIGURE 7

$^1$H-NMR spectrum of the YL-01641P-$B_2$

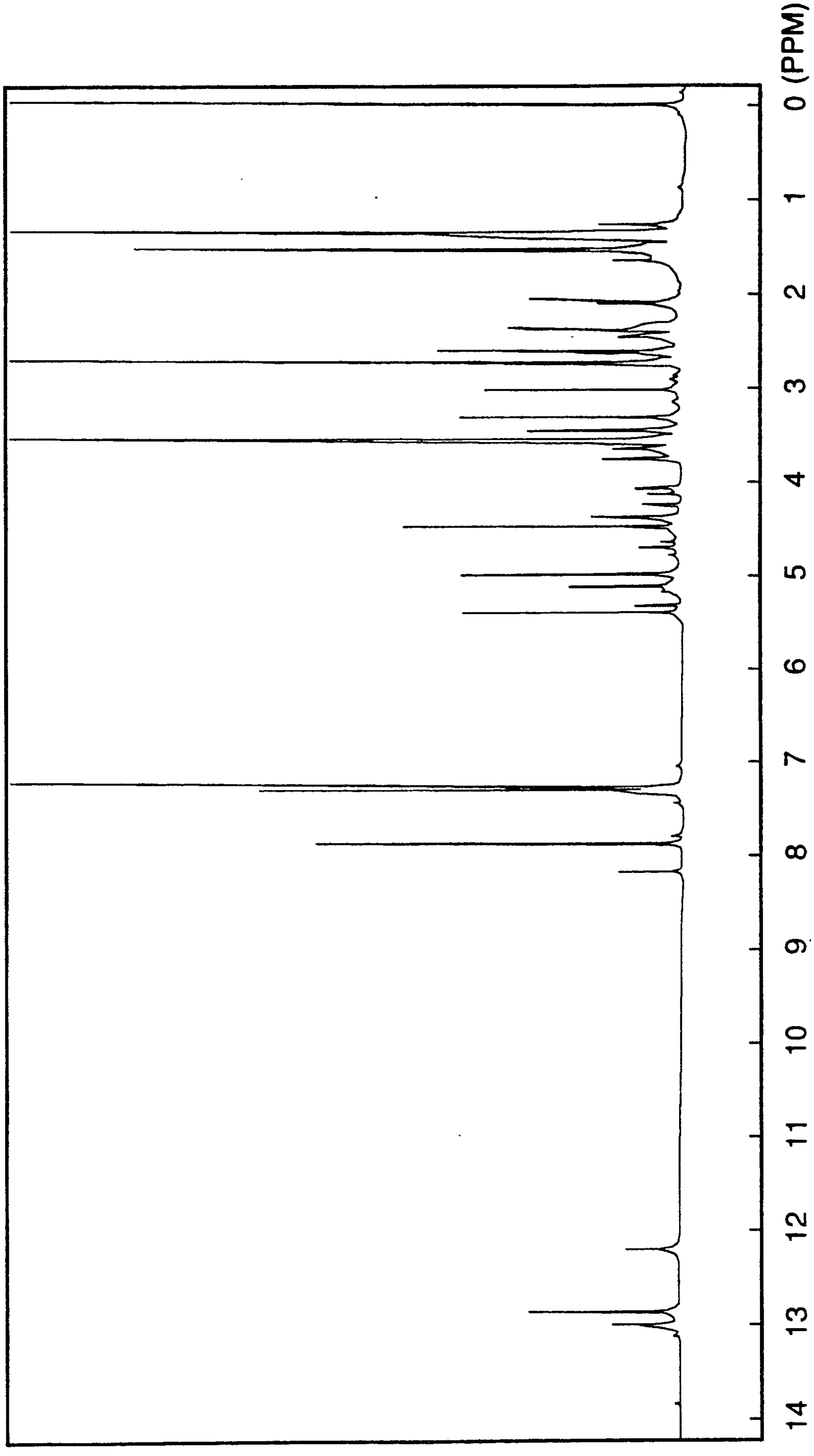

# FIGURE 8

Ultraviolet absorption spectrum of YL-01641P-$B_1$

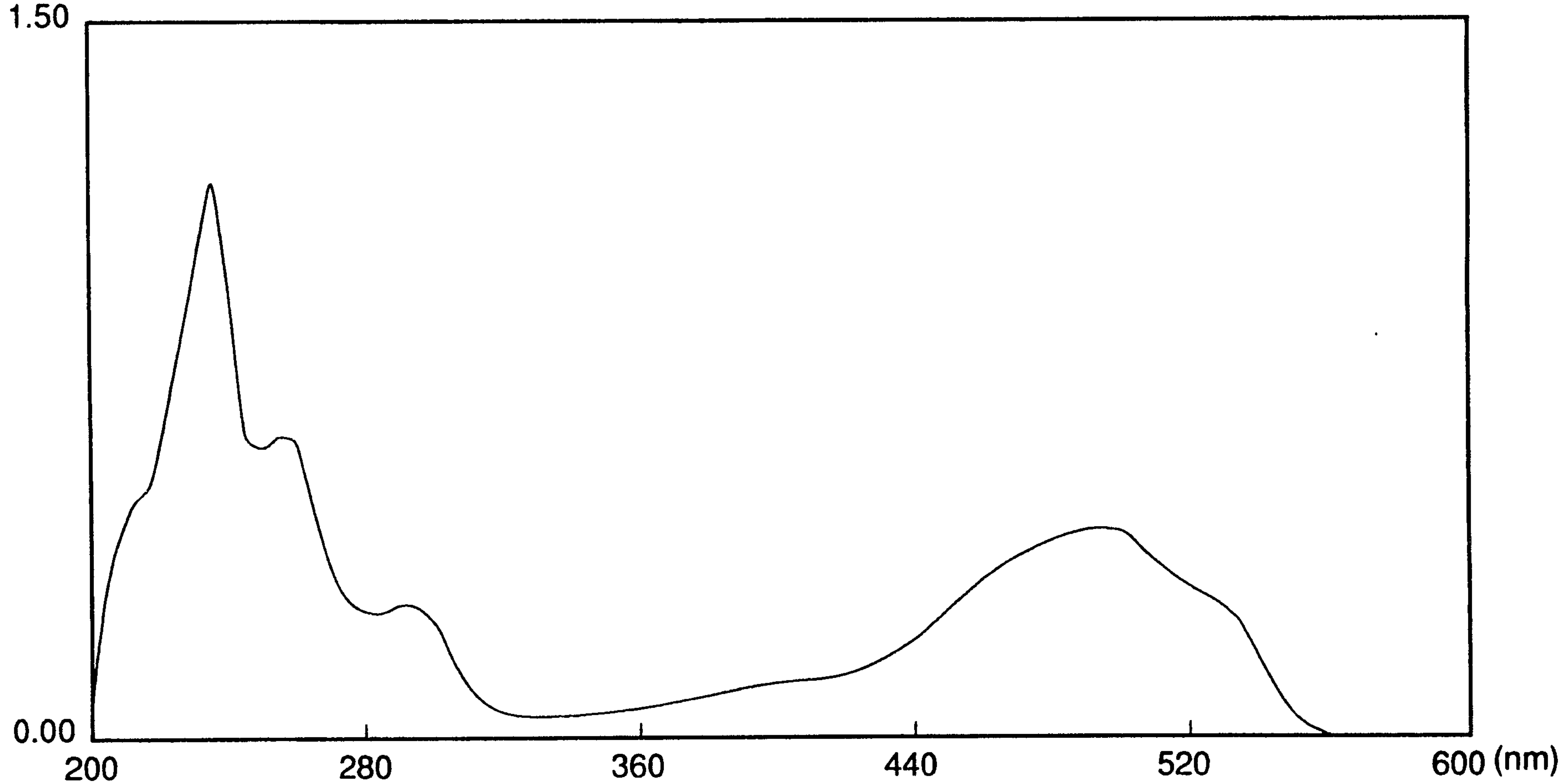

FIGURE 9

H-NMR spectrum of YL--01641-$B_1$

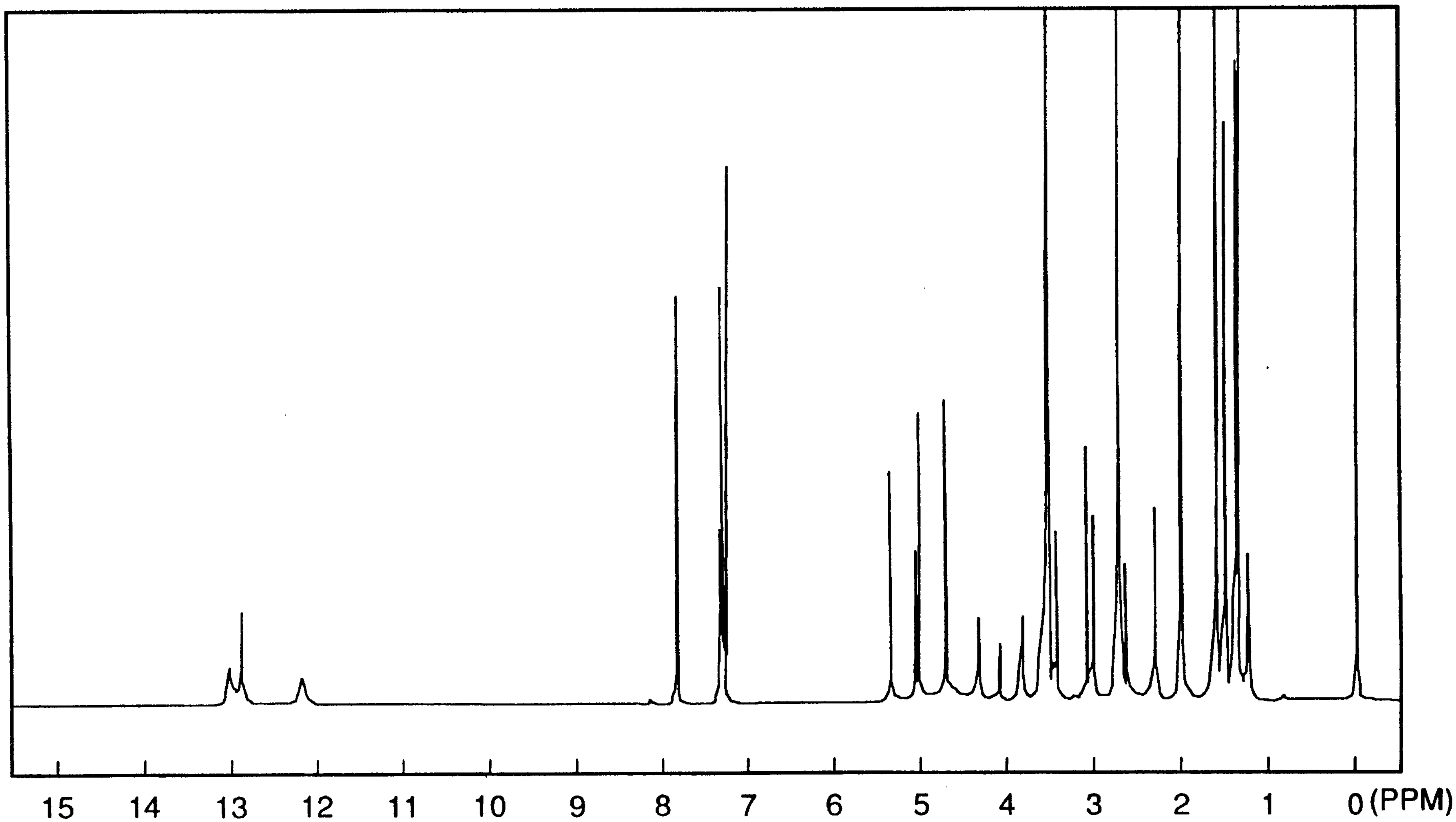

FIGURE 10

$^{13}C$-NMR spectrum of YL-01641P-$B_1$

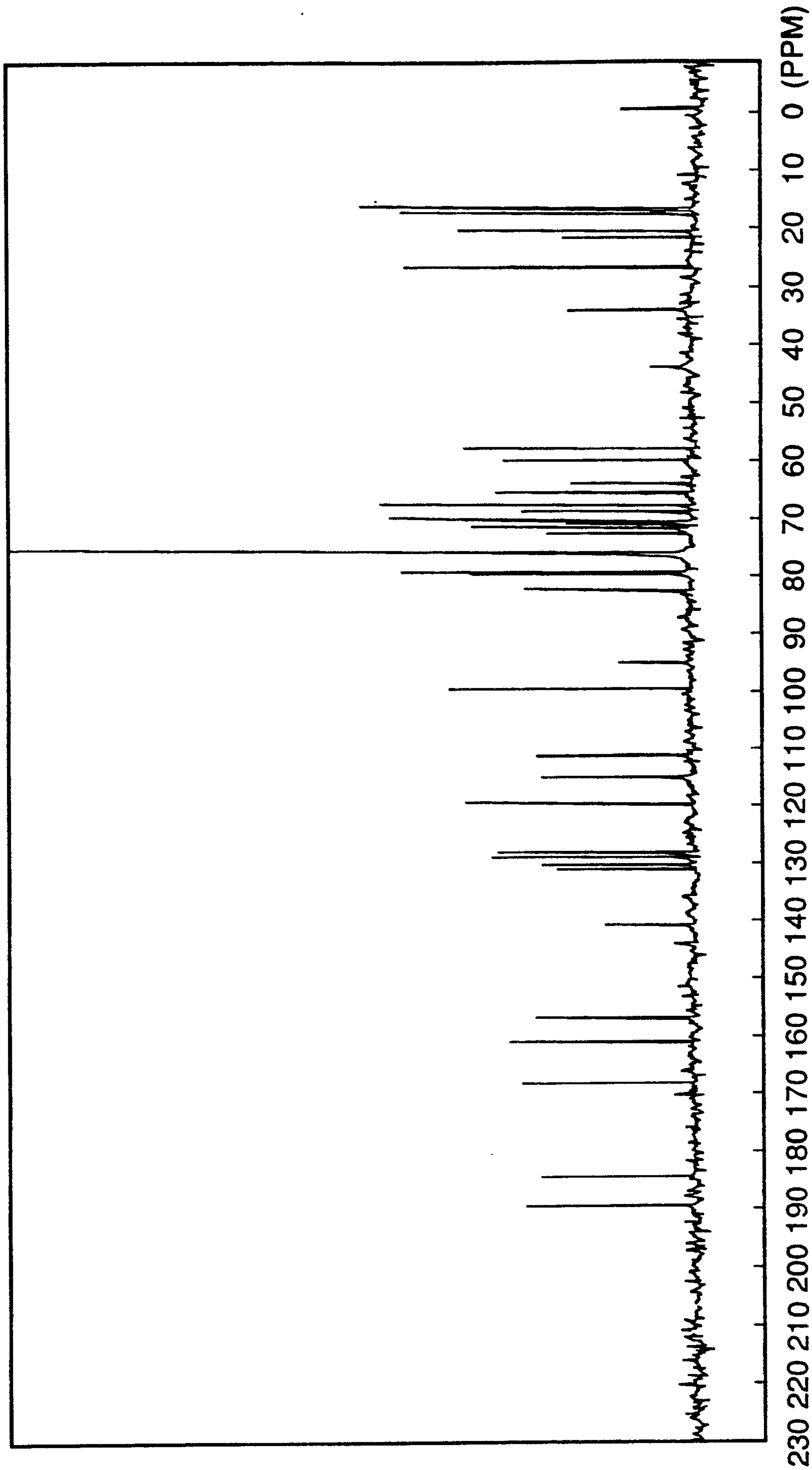

# FIGURE 11

Ultravioret absorption spectrum of YL-01641P-C 1

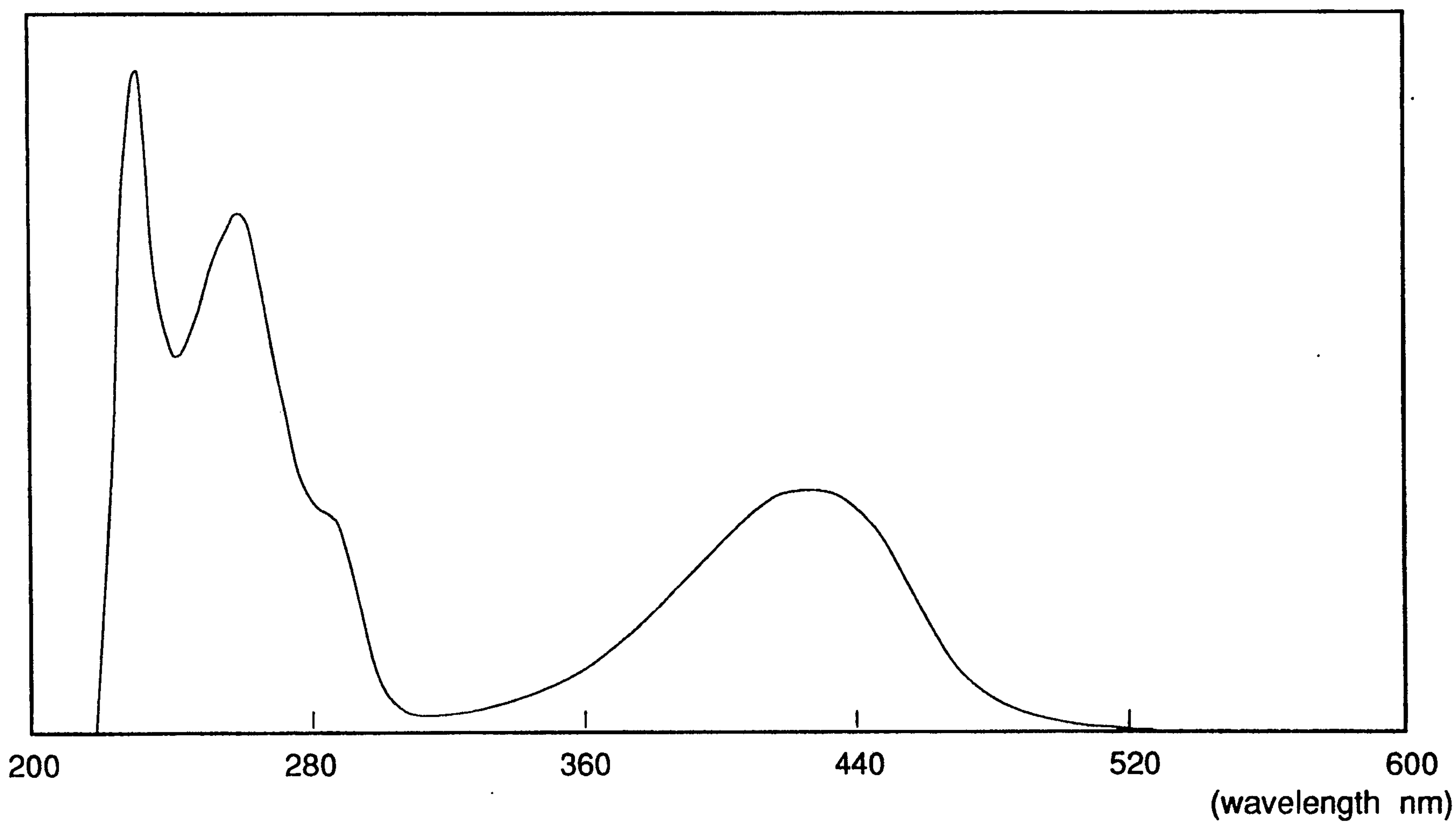

FIGURE 12

$^{1}H$-NMR spectrum of YL-01641P-$C_1$